# EUROPEAN PATENT APPLICATION

(11) **EP 4 725 955 A1**
(43) Date of publication of application: **15.04.2026**
(21) Application number: 24830497.4
(22) Date of filing: 12.06.2024
(51) Int. Cl.: C07K 7/06, A61K 38/08, A61K 47/64, A61P 35/00

(54) **PEPTIDE TARGETING C-MET, PHARMACEUTICAL COMPOSITION COMPRISING SAID PEPTIDE AND USE THEREOF**

(30) Priority: 25.06.2023 WO PCT/CN2023/102222
(71) Applicant: Nanjing Anji Biotechnology Co., Ltd., Nanjing, Jiangsu 210000 (CN)
(72) Inventor: XU, Hanmei, Nanjing, Jiangsu 210000 (CN); HAO, Chaowei, Nanjing, Jiangsu 210000 (CN); CHEN, Peng, Nanjing, Jiangsu 210000 (CN)
(74) Representative: Prüfer & Partner mbB Patentanwälte · Rechtsanwälte
(86) International application number: PCT/CN2024/098752
(87) International publication number: WO 2025/001851

(57) **Abstract**

A peptide targeting c-Met, derivatives thereof, a pharmaceutical composition and drug-peptide conjugate comprising the peptide or the derivatives thereof, and the use of same in the detection, prevention or treatment of diseases (e.g., cancers). In addition, the peptide or the derivatives thereof can increase the water solubility of a drug.

## Description

### CROSS-REFERENCE TO RELATED APPLICATION

This application claims the priority of application PCT/CN2023/102222 filed on June 25, 2023. The prior application is deemed a part of the disclosure of the present application and incorporated herein in its entirety.

### TECHNICAL FIELD

The present disclosure relates to the field of medicines, and particularly to a peptide targeting c-Met, a conjugate and pharmaceutical composition comprising the peptide, and uses.

### BACKGROUND

As a leading global killer, cancer is endangering the lives of a growing number of people. According to data released by China's Ministry of Health, cancer has surpassed cardiovascular diseases to become the leading cause of death among Chinese residents, both in urban and rural areas.

In recent decades, with the development of the medical and health fields, remarkable progress has been made in the treatment and control and other aspects of cancer, and various drugs and treatment methods have been constantly emerging. However, surgical treatment, chemotherapy and radiotherapy are currently still primary three treatment means, and these treatment means are difficult to achieve satisfactory therapeutic effects. The common drug therapy has important effects, but such treatment means often has huge damage to normal tissues and cells. The anti-cancer drug market is growing rapidly, in which targeted anti-cancer drugs are growing at the fastest rate. The targeted drugs comprise common small-molecule target drugs, monoclonal antibodies and the like. They deliver the drugs to cancer sites by taking cancer cells or tissues as targets, thereby reducing the damage to normal tissues and normal cells and then achieving the purpose of specific treatment.

c-Met, also called a hepatocyte growth factor receptor (HGFR), is one of receptor tyrosine kinase family members. c-Met is over-expressed in primary epithelial carcinomas such as breast cancer, colon cancer, gastric cancer, kidney cancer, liver cancer, lung cancer and pancreatic cancer, with an increase range in expression being 35-65% for breast cancer, 30-70% for gastric cancer, 25-60% for pancreatic cancer, 25-60% for hepatocellular carcinoma, 15-80% for renal cell carcinoma and 35-70% for non-small cell lung cancer (Biochim Biophys Acta Rev Cancer. 2020, 1874, 188425.). In various cancers, c-Met can activate a downstream signaling pathway, promote the proliferation and migration of cancer cells and motivate cancer invasion, metastasis and angiogenesis after binding to a ligand hepatocyte growth factor (HGF). Therefore, cancer therapy and imaging taking c-Met as a target have become hot research directions.

At present, blocking the signaling of c-Met-HGF is one of the effective means to treat cancers. This means can inhibit the specific binding of c-Met to HGF by using a small-molecule kinase inhibitor, an antibody and a peptide. Compared with small-molecule drugs or antibody drugs, the peptide has the characteristics of high affinity, almost no immunogenicity, low production cost, easy modification and the like. The existing technology (for example, CN112979753B) has disclosed a peptide targeting c-Met, however, its application in clinical therapy and diagnosis is limited due to its poor solubility in water and the need for improved affinity to c-Met. Therefore, it is urgent to develop an improved peptide targeting c-Met, such as a peptide targeting c-Met with better water solubility and/or higher affinity to c-Met.

### SUMMARY

Based on the above description, the present disclosure provides a peptide targeting c-Met, which is simple in structure and easy to synthesize, separate and purify. The peptide exhibits specific binding to a c-Met protein in a subject both in vitro and in vivo. The present disclosure further provides a peptide having a good ability of targeting a c-Met protein in the subject. The peptide is enriched in cancer tissues highly expressing c-Met. The present disclosure further provides the above-mentioned peptide targeting c-Met, which has improved affinity to c-Met and/or water solubility. The peptide of the present disclosure also exhibits improved drug solubility after conjugation with a drug.

In a first aspect of the present disclosure, provided is a peptide targeting c-Met or a derivative thereof, comprising the following formula I or formula II,

(R)-(X 1)-(X2)-(X3)-(X4)-(X5)-(X6) Formula I

(Y1)-(Y2)-(Y3)-(Y4)-(A)-(N)-(Y5) Formula II

wherein, X1-X6 each independently represent any one amino acid or a derivative thereof; Y1-Y5 each independently represent any one amino acid or a derivative thereof, and the formula II does not comprise the sequence (D)-(Q)-(I)-(I)-(A)-(N)-(N) of CM7-1; and the peptide or derivative thereof has similar or better binding affinity to c-Met relative to CM7-1.

In one embodiment of the present disclosure, in the formula I, X1 represents S, H or E, X2 represents P, I or D, X3 represents any one amino acid or a derivative thereof, X4 represents A, M or C, X5 represents N, C or P, and X6 represents any one amino acid or a derivative thereof; in the formula II, Y1 represents any one amino acid or a derivative thereof, Y2 represents Q or A, Y3 represents I or A, Y4 represents I or A, Y5 represents N or A, and the formula II does not comprise (D)-(Q)-(I)-(I)-(A)-(N)-(N).

In one embodiment of the present disclosure, the peptide targeting c-Met or derivative thereof comprises any one sequence selected from SEQ ID NO:1, SEQ ID NOs:3-6, SEQ ID NO:8, SEQ ID NOs:9-144 and SEQ ID NOs:148-150.

In one embodiment of the present disclosure, the peptide targeting c-Met or derivative thereof comprises any one sequence selected from SEQ ID NO:1, SEQ ID NOs:3-6, SEQ ID NO:8, SEQ ID NOs:9-20, SEQ ID NO:22, SEQ ID NO:28, SEQ ID NO:35, SEQ ID NO:40, SEQ ID NO:48, SEQ ID NO:64, SEQ ID NO:66, SEQ ID NO:70, SEQ ID NO:72, SEQ ID NOs:79-81, SEQ ID NOs:83-85, SEQ ID NO:90, SEQ ID NO:96, SEQ ID NO:105, SEQ ID NO:111, SEQ ID NO:123, SEQ ID NO:125, SEQ ID NO:135, SEQ ID NO:144 and SEQ ID NOs:148-150.

In one embodiment of the present disclosure, the peptide targeting c-Met or derivative thereof comprises the formula I,

(R)-(X1)-(X2)-(X3)-(X4)-(X5)-(X6) Formula I

wherein, X1 is S, X2 is P, X3-6 each independently represent any one amino acid or a derivative thereof, and the peptide or derivative thereof has similar or better binding affinity to c-Met relative to P1-AI (SEQ ID NO:1, RSPYANS).

In one embodiment of the present disclosure, the peptide targeting c-Met or derivative thereof comprises any one sequence selected from SEQ ID NOs: 148-150.

In a second aspect of the present disclosure, provided is a peptide targeting c-Met or a derivative thereof, comprising the following formula III,

(R)-(S)-(P)-(X7)-(X8)-(X9)-(X10) Formula III

wherein, X7-10 each independently represent any one amino acid or a derivative thereof; and the peptide or derivative thereof has similar or better binding affinity to c-Met relative to P1-AI.

In one embodiment of the present disclosure, the peptide targeting c-Met or derivative thereof comprises any one sequence selected from SEQ ID NOs: 148-150.

In a third aspect of the present disclosure, provided is an isolated polynucleotide encoding the peptide or derivative thereof according to the present disclosure.

In a fourth aspect of the present disclosure, provided is an expression vector expressing the polynucleotide according to the present disclosure.

In a fifth aspect of the present disclosure, provided is a host cell, comprising the polynucleotide according to the present disclosure or the expression vector according to the present disclosure.

In a sixth aspect of the present disclosure, provided is a drug-peptide conjugate, comprising the peptide or derivative thereof of the present disclosure, and a drug. In some embodiments, the peptide or derivative thereof of the present disclosure is conjugated with the drug directly (e.g., by a covalent bond) or indirectly (e.g., by a linker).In some preferred embodiments, the drug is selected from compounds such as cytotoxic agents, including but not limited to, at least one of camptothecin, docetaxel and paclitaxel.

In a seventh aspect of the present disclosure, provided is a pharmaceutical composition, comprising the peptide or derivative thereof, the polynucleotide, the expression vector, the host cell or the drug-peptide conjugate according to the present disclosure, and a pharmaceutically acceptable vector or salt thereof.

In an eighth aspect of the present disclosure, provided is use of the peptide or derivative thereof of the present disclosure for increasing the water solubility of a drug. In one embodiment, the peptide or derivative thereof of the present disclosure is conjugated with the drug directly (e.g., by a covalent bond) or indirectly (e.g., by a linker). In some preferred embodiments, the drug is selected from compounds such as cytotoxic agents, including but not limited to, at least one of camptothecin, docetaxel and paclitaxel.

In a ninth aspect of the present disclosure, provided is a method for increasing the water solubility of a drug, comprising conjugating the peptide or derivative thereof of the present disclosure with the drug directly (e.g., by a covalent bond) or indirectly (e.g., by a linker). In some preferred embodiments, the drug is selected from compounds such as cytotoxic agents, including but not limited to, at least one of camptothecin, docetaxel and paclitaxel.

In a tenth aspect of the present disclosure, provided is use of the peptide or derivative thereof, the polynucleotide, the expression vector, the host cell, the drug-peptide conjugate or the pharmaceutical composition according to the present disclosure in the preparation of a reagent or drug for detecting, preventing or treating a disease.

In an eleventh aspect of the present disclosure, provided is a method for detecting, preventing or treating a disease, the method comprising administrating to a subject in need thereof a detectably or preventively or therapeutically effective amount of the peptide or derivative thereof, the polynucleotide, the expression vector, the host cell, the drug-peptide conjugate or the pharmaceutical composition according to the present disclosure.

### BRIEF DESCRIPTION OF THE DRAWINGS

Next, the brief description of drawings will be provided and used for illustrating the exemplary embodiments disclosed in the present disclosure, but not limiting these embodiments.
FIG. 1 shows a structure of a polypeptide P1-AI.
FIG. 2A shows high performance liquid chromatography (HPLC) detection results of P1-AI, FITC-P1-AI, CM7-1, FITC-CM7-1, FITC-CM7-1.1 and FITC-CM7-1.2 synthesized in example 1.
FIG. 2B shows HPLC detection results of FITC-CM7-1.3, FITC-CM7-1.4, FITC-CM7-1.5, FITC-CM7-1.6 and Cy5-P1-AI synthesized in example 1.
FIG. 3 shows mass spectrometry analysis results of P1-AI, FITC-P1-AI, Cy5-P1-AI, CM7-1, FITC-CM7-1, FITC-CM7-1.1, FITC-CM7-1.2, FITC-CM7-1.3, FITC-CM7-1.4, FITC-CM7-1.5 and FITC-CM7-1.6 synthesized in example 1.
FIG. 4 shows flow cytometry detection results of c-Met expression in different cells in example 2, wherein the horizontal axis represents a fluorescence signal, and the longitudinal axis represents the quantity of cells. In the figure, a light-colored peak represents c-Met, and a dark-colored peak represents FITC-c-Met isotype control IgG.
FIG. 5 shows a binding curve of the peptide of the present disclosure and c Met positive cell MKN-45 in example 3, wherein the horizontal axis represents log (polypeptide concentration), and the concentration unit is µM; and the longitudinal axis represents a binding rate (%).
FIG. 6 shows a binding curve of the peptide of the present disclosure and c-Met positive cell MKN-45 in example 3, wherein the horizontal axis represents a fluorescence signal, and the longitudinal axis represents the quantity of cells. In the figure, the leftmost curve represents a blank control group without a peptide, the middle curve is FITC-CM7-1, and the rightmost curve is FITC-P1-A.
FIG. 7 is a molecule docking diagram of polypeptide CM7 and polypeptide P1-AI in example 4, showing their docking scores and average values of hydrophobicity.
FIG. 8 shows a binding curve of the peptide of the present disclosure and human hepatic stellate cell LX-2 that barely expresses c-Met in example 5, wherein the horizontal axis represents a fluorescence signal, and the longitudinal axis represents the quantity of cells.
FIG. 9 shows water-solubility detection results of the peptide of the present disclosure in example 6.
FIG. 10 shows detection results of interaction between the peptide of the present disclosure and a c-Met protein using microscale thermophoresis (MST) in example 7, wherein the horizontal axis represents the concentration of the c-Met protein, and the unit is µM; and the longitudinal axis represents fluorescence intensity.
FIG. 11 shows a living imaging result of Cy5-P1-AI in tumor-bearing mice in example 8.
FIG. 12 shows the result of peptides P1-AI, P1-AI-1, P1-AI-2, P1-AI-3, P1-AI-4, P1-Al-6 and P1-AI-7 of the present disclosure binding to c-Met positive cell MKN-45 in example 9, wherein the horizontal axis represents a fluorescence signal; and the longitudinal axis represents the quantity of cells, a dark color represents a fluorescence chemical shift after addition of a polypeptide, and a light color represents a blank control without a peptide.
FIG. 13 shows a concentration-peak area standard curve of CPT, CPT-P1-AI, DOC, DOC-P1-AI, PTX and PTX-P1-AI series standard solutions in example 10, wherein the horizontal axis represents a concentration; and the longitudinal axis represents a peak area.

### DETAILED DESCRIPTION OF EMBODIMENTS

Unless otherwise stated, all numbers which are used in this specification and claims representing content, concentration, ratio, weight, particle size, percentage, technical effect and the like shall be understood as being modified by the terms "about" or "approximately". Therefore, unless indicated to the contrary, the numerical parameters set forth in the following specification and appended claims are approximate values.

Unless otherwise specified, the terms used herein have ordinary meanings understood by those skilled in the art. Those skilled in the art may vary depending on the desired properties and effects sought through the present application, and each numerical parameter shall be construed in accordance with the number of significant figures, conventional rounding methods, or as understood by those skilled in the art. In general, nomenclatures used herein and the experimental procedures described for organic chemistry, medicinal chemistry and biology are well known in the art, and commonly employed in the art. Unless otherwise defined, all technical and scientific terms used herein generally have the same meanings as those commonly understood by persons of ordinary skill in the art to which the present application pertains. In cases where a term used herein has multiple definitions, unless otherwise stated, the definition set forth in this section shall prevail.

As used herein, the expression "A and/or B" includes three scenarios: (1) A; (2) B; and (3) both A and B. The expression "A, B, and/or C" includes seven scenarios: (1) A; (2) B; (3) C; (4) both A and B; (5) both A and C; (6) both B and C; and (7) A, B and C. The meanings of similar expressions may be deduced by analogy.

As used herein, the term "each independently" means that multiple events have no influence on one another. For example, the sentence "X and Y are each independently selected from any one of a, b, c, d, e, f and g" means that X may be any one of a, b, c, d, e, f and g, and Y may also be any one of a, b, c, d, e, f and g. The selection of X and the selection of Y may be the same or different, both of which are independent of each other without mutual interference.

As used herein, alanine scanning (Cunningham and Wells, Science 244, 1081-1085, 1989) is used for identifying amino acids that play a key role in the activity of the peptide of the present disclosure (e.g., a binding affinity to an acetylcholine receptor), such that these amino acids are not substituted. By alanine scanning, a mutation introduced into each residue of this molecule and the biological activity of the resulting molecule is inspected to identify amino acid residues that are critical to the activity of the molecule.

As used herein, the terms "include" and "comprise" means that other elements are not excluded besides the listed elements.

### Peptide or derivative thereof

The present application provides a peptide targeting c-Met or a derivative thereof, comprising the following formula I or formula II,

(R)-(X1)-(X2)-(X3)-(X4)-(X5)-(X6) Formula I

(Y1)-(Y2)-(Y3)-(Y4)-(A)-(N)-(Y5) Formula II

wherein, X1-X6 each independently represent any one amino acid or a derivative thereof; Y1-Y5 each independently represent any one amino acid or a derivative thereof, and the formula II does not comprise the sequence (D)-(Q)-(I)-(I)-(A)-(N)-(N) of CM7-1; and the peptide or derivative thereof has similar or better binding affinity to c-Met relative to CM7-1.

In some embodiments, X1 in the formula I represents S, H or E. In some embodiments, X2 in the formula I represents P, I or D. In some embodiments, X3 in the formula I represents any one amino acid or a derivative thereof. In some embodiments, X4 in the formula I represents A, M or C. In some embodiments, X5 in the formula I represents N, C or P. In some embodiments, X6 in the formula I represents any one amino acid or a derivative thereof.

In some embodiments, Y1 in the formula II represents any one amino acid or a derivative thereof. In some embodiments, Y2 in the formula II represents Q or A. In some embodiments, Y3 in the formula II represents I or A. In some embodiments, Y4 in the formula II represents I or A. In some embodiments, Y5 in the formula II represents N or A. In some embodiments, the formula II does not comprise (D)-(Q)-(I)-(I)-(A)-(N)-(N).

In some embodiments, the peptide targeting c-Met or derivative thereof comprises any one sequence selected from SEQ ID NO:1, SEQ ID NOs:3-6, SEQ ID NO:8, SEQ ID NOs:9-144 and SEQ ID NOs:148-150.

In some embodiments, the peptide targeting c-Met or derivative thereof comprises any one sequence selected from SEQ ID NO:1, SEQ ID NOs:3-6,SEQ ID NO:8, SEQ ID NOs:9-20, SEQ ID NO:22, SEQ ID NO:28, SEQ ID NO:35, SEQ ID NO:40, SEQ ID NO:48, SEQ ID NO:64, SEQ ID NO:66, SEQ ID NO:70, SEQ ID NO:72, SEQ ID NOs:79-81, SEQ ID NOs:83-85, SEQ ID NO:90, SEQ ID NO:96, SEQ ID NO:105, SEQ ID NO:111, SEQ ID NO:123, SEQ ID NO:125, SEQ ID NO:135, SEQ ID NO:144 and SEQ ID NOs:148-150.

In some embodiments, the sequence of the peptide targeting c-Met or derivative thereof comprises SEQ ID NO:1. In some embodiments, the sequence of the peptide targeting c-Met or derivative thereof comprises SEQ ID NO:10. In some embodiments, the sequence of the peptide targeting c-Met or derivative thereof comprises SEQ ID NO:90. In some embodiments, the sequence of the peptide targeting c-Met or derivative thereof comprises SEQ ID NO:144.

In some embodiments, the peptide targeting c-Met or derivative thereof comprises the following formula I,

(R)-(X1)-(X2)-(X3)-(X4)-(X5)-(X6) Formula I

wherein, X1 is S, X2 is P, X3-6 each independently represent any one amino acid or a derivative thereof, and the peptide or derivative thereof has similar or better binding affinity to c-Met relative to P1-AI (SEQ ID NO:1, RSPYANS).In some embodiments, the peptide or derivative thereof comprises any one sequence selected from SEQ ID NOs:148-150.

In some embodiments, compared with the peptide, the derivative comprises any one or more modifications selected from amino acid modification, conservative amino acid substitution and hydrogen substitution in amino acid residue.

As used herein, the term "peptide" refers to a compound formed by linking amino acids via a peptide bond. A peptide composed of three or more amino acid molecules is a polypeptide. As used herein, unless otherwise stated or contradictory to the context, the term "peptide" encompasses the referenced peptide itself, as well as pharmaceutically acceptable salts, prodrugs and metabolites thereof. The peptide of the present application is generated by using conventional technologies in the art, including but not limited to a solid-phase synthesis method (e.g., a Fmoc peptide synthesis method) and a liquid-phase synthesis method.

As used herein, the term "prodrugs" refers to all molecules that are converted into the peptide or derivative thereof of the present disclosure under physiological conditions in vivo for example through oxidation, reduction, hydrolysis and other reactions performed under the actions of enzymes, stomach acids and the like.

As used herein, the term "metabolites" refers to all molecules that are derived from the peptide or derivative thereof of the present disclosure in cells or organisms (preferably human).

Herein, when the peptide is used, "amino acid" and "amino acid residue" have the same meaning, which means that when amino acids are linked via a chemical bond, some moieties are lost because of involving in the formation of a linkage bond, and the remaining amino acid moieties are amino acid residues.

As used herein, unless otherwise stated or contradictory to the context, "amino acid" encompasses L-type amino acids and D-type amino acids.

As used herein, "polar amino acids" refer to Glycine (G), Tyrosine (Y), Serine (S), Cysteine (C), Asparagine (N), Glutamine (Q), Threonine (T), Aspartic acid (D), Glutamic acid (E), Lysine (K), Arginine (R) and Histidine (H).

As used herein, the "derivative" of the peptide refers to a product that is obtained by amino acid modification, conservative amino acid substitution and/or hydrogen substitution in amino acid residue performed on the basis of the referenced peptide.

As used herein, the types of the term "amino acid modification" include, but are not limited to, N-terminal modification, C-terminal modification and side-chain modification. The methods of "amino acid modification" include, but are not limited to, hydroxylation, carboxylation, alkylation, acylation, phosphorylation, sulfonation, amidation, aldehydation, alcoholation, mercaptoethylamination, esterification, glycosylation and other modifications.

As used herein, the term "conservative amino acid substitution" refers to substitution of one amino acid with another amino acid having a similar structure and/or chemical properties, for example, substitution of leucine with isoleucine or valine, substitution of aspartic acid with glutamic acid, or substitution of threonine with serine. Therefore, "conservative amino acid substitution" means that an amino acid that is not crucial to the activity of the peptide is substituted, or one amino acid is substituted with other amino acids having similar properties (e.g., acidic, basic, positively charged or negatively charged, polar or non-polar, etc.), such that the activity of the peptide is not reduced even if a critical amino acid is substituted. The conservative amino acid substitution providing amino acids with similar functions are well-known in the art. For example, the following six groups each contain amino acids that are conservative substitutions of one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y) and Tryptophan (W).

As used herein, the hydrogen substitution in amino acid residue may be a fact that hydrogen in amino acid residue is substituted by any conventional substituent known in the art, including but not limited to deuterated, alkyl, alkenyl, alkynyl, aryl, alkoxy, carboxyl, aldehyde, carbonyl, hydroxyl, halogen, cyano, acyl, sulfo, amino, mercapto or nitro.

The present application provide a peptide targeting c-Met or a derivative thereof, comprising the following formula III,

(R)-(S)-(P)-(X7)-(X8)-(X9)-(X10) Formula III

wherein, X7-10 each independently represent any one amino acid or a derivative thereof; and the peptide or derivative thereof has similar or better binding affinity to c-Met relative to P1-AI.

In some embodiments, the peptide targeting c-Met or derivative thereof comprises any one sequence selected from SEQ ID NOs: 148-150. In some embodiments, the peptide targeting c-Met or derivative thereof comprises SEQ ID NO: 148.

In some embodiments, compared with the peptide, the derivative comprises any one or more modifications selected from amino acid modification, conservative amino acid substitution and hydrogen substitution in amino acid residue.

### Polynucleotide, expression vector and host cell

The present application provides an isolated polynucleotide encoding the peptide or derivative thereof of the present application.

As used herein, "polynucleotide" refers to a polymer of nucleotides (nucleotide or deoxynucleotide ) with any lengths. This noun refers to a primary structure of a molecule. Therefore, it comprises not only double-stranded and single-stranded RNA, but also double-stranded and single-stranded DNA. It also comprises polynucleotides which are subjected to methylation and/or capping modification, and unmodified polynucleotides. The polynucleotide described herein is not necessarily obtained by a physical method, and may also be produced in any manner, including chemical synthesis, DNA replication, reverse transcription or transcription, etc.

The present application provides an expression vector expressing the polynucleotide of the present application.

The present application provides a host cell, comprising the polynucleotide of the present application or the expression vector of the present application. In some embodiments, the host cell is selected from at least one of Escherichia coli cells, Saccharomyces cerevisiae cells, Bacillus subtilis cells, and animal cells such as Chinese hamster ovary (CHO) cells and human embryonic kidney (HEK) cells.

As used herein, "host cell" refers to a cell that serves as an expression vector or a recipient for other transformed genetic fragments. They comprise original cells that have been transfected and their progenies. The host cell comprises prokaryotic cells, yeast cells or other eukaryotic cells.

### Drug-peptide conjugate

The present application provides a drug-peptide conjugate, comprising the peptide or derivative thereof of the present disclosure, and a drug. In some embodiments, the peptide or derivative thereof of the present disclosure is conjugated with the drug directly (e.g., by a covalent bond) or indirectly (e.g., by a linker). In some embodiments, the linker is selected from at least one of a cleavable linker and a non-cleavable linker. In some embodiments, the linker comprises ((G)n(S))m, wherein n is an integer of 1-8, and m is an integer of 1-8. In some embodiments, the linker comprises 1-8 amino acids, such as Val-Cit and Glu-Val-Cit. In some embodiments, the linker comprises CO(O)C(CH2)ₙC(O)OH, wherein n is an integer of 2-8. In some embodiments, the linker comprises at least one of succinic acid, fumaric acid, glutaric acid, adipic acid, pimelic acid and suberic acid.

In some embodiments, the drug is selected from at least one of a small-molecule drug, an antibody, a polypeptide, a protein, a chemical drug, a chemotherapy drug and a fluorescent label. In some embodiments, the drug includes, but is not limited to, cytotoxic agents which can be any compound leading to cell death or inducing cell death, or reducing cell vitality in a certain manner. Exemplary cytotoxic agents comprise for example maytansine and maytansine analogs, taxanes (e.g., paclitaxel and docetaxel), CC-1065 and CC-1065 analogs, dolastatin and dolastatin analogs, methotrexate, daunorubicin, doxorubicin, vincristine, vinblastine, melphalan, mitomycin C, chlorambucil, calicheamicin, microtubulomycin and microtubulomycin analogs, and duocarmycin and duocarmycin analogs. In some preferred embodiments, the drug is selected from at least one of cytotoxic agents such as camptothecin, docetaxel and paclitaxel.

### Pharmaceutical composition

The present application provides a pharmaceutical composition, comprising the peptide or derivative thereof and the drug-peptide conjugate according to the present disclosure, and a pharmaceutically acceptable carrier and salt thereof. The present application provides a pharmaceutical composition, comprising the peptide or derivative thereof, the polynucleotide, the expression vector, the host cell or the drug-peptide conjugate according to the present disclosure, and a pharmaceutically acceptable carrier and salt thereof.

As used herein, the term "pharmaceutical composition" refers to a mixture of the peptide of the present disclosure and other chemical components such as a carrier, a stabilizer, a diluent, a disperser, a suspending agent, a thickener and/or an excipient. The pharmaceutical composition is conducive to administrating the peptide to an organism. There are various technologies for administrating the peptide in the art, including but not limited to subcutaneous injection, intramuscular injection, intravenous injection, intraperitoneal injection, intrathecal injection, oral administration, transdermal administration, pulmonary administration, ophthalmic administration and topical administration.

In the present application, the pharmaceutical composition can be formulated into a dosage form that is suitable for administration into a subject through a required administration route. The dosage form includes, but is not limited to, tablets, capsules, caplets, pills, lozenges, powders, syrups, elixirs, suspensions, solutions, emulsions, transdermal patches, suppositories, inhalants, creams, ointments, lotions, pastes, sprays, lyophilizates, injections and gels.

As used herein, the term "pharmaceutically acceptable salt" includes acid addition salts and base addition salts. An appropriate acid addition salt is formed by an acid forming a non-toxic salt. The examples of the acid addition salt include, but are not limited to, acetates, adipates, aspartates, benzoates, besylates, bicarbonates/carbonates, bisulfates/sulfates, borates, camphorsulfonates, citrates, cyclohexylamine sulfonates, edisylates, formates, fumarates, glucoheptonates, gluconates, glucuronates, hexafluorophosphates, 2-(4-hydroxybenzyl)benzoates, hydrochlorides/chlorides, hydrobromides/bromides, hydriodides/iodides, 2-isethionates, lactates, malates, maleates, malonates, mesylates, methylsulfates, naphthalenecarboxylates, 2-naphthalenesulfonates, nicotinates, nitrates, orotates, oxalates, palmitates, phosphates/hydrogen phosphates/dihydrogen phosphates, pyroglutamates, glucarates, stearates, salicylates, tannates, tartrates, tosylates and trifluoroacetates. An appropriate base addition salt is formed by a base forming a non-toxic salt. The examples of the base addition salt include, but are not limited to, aluminum, arginine, calcium, choline, diethylamine, diethanolamine, glycine, lysine, magnesium, meglumine, ethanolamine, potassium, sodium, tromethamine and zinc salts. Hemisalts of acids and bases can also be formed, such as hemisulfates and hemicalcium salts. A review about appropriate salts refers to Handbook of Pharmaceutical Salts: Properties, Selection and Use by Stahl and Wermuth (Wiley-VCH, 2002).

The term "pharmaceutically acceptable carrier" includes pharmaceutically acceptable materials, compositions or carriers, for example, liquid or solid fillers, diluents, excipients, solvents or encapsulation materials, which involves in carrying or transporting the peptide of the present disclosure in a subject, or carrying or transporting the peptide of the present disclosure to the subject, so that its expected functions can be implemented. Each salt or carrier must be "acceptable" in the sense of being compatible with other components of a formulation, and must not be harmful to the subject. Some examples of materials that can serve as the pharmaceutically acceptable carriers include: sugar, such as lactose, glucose and sucrose; starch, such as corn starch and potato starch; celluloses and derivatives thereof, such as sodium carboxymethylcellulose, ethylcellulose and cellulose acetate; powdered tragacanth; malt; gelatin; talc; excipients, such as cocoa butter and suppository wax; oil, such as peanut oil, cottonseed oil, safflower oil, sesame oil, olive oil, corn oil and soybean oil; glycol, such as propylene glycol; polyol, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethanol; phosphate buffer solutions; diluents; granulating agents; lubricants; binders; disintegrants; wetting agents; emulsifiers; colorants; mold release agents; coating agents; sweeteners; flavoring agents; perfuming agents; preservatives; antioxidants; plasticizers; gelling agents; thickeners; hardeners; setting agents; suspending agents; surfactants; humectants; carriers; stabilizers; and other non-toxic compatible substances used in a pharmaceutical formulation, or any combinations thereof.

### Use and method

The present application provides use of the peptide or derivative thereof of the present disclosure for increasing the water solubility of a drug. In one embodiment, the peptide or derivative thereof of the present disclosure is conjugated with the drug directly (e.g., by a covalent bond) or indirectly (e.g., by a linker).

The present application provides a method for increasing the water solubility of a drug, comprising conjugating the peptide or derivative thereof of the present disclosure with the drug directly (e.g., by a covalent bond) or indirectly (e.g., by a linker).

In some embodiments, the linker is selected from at least one of a cleavable linker and a non-cleavable linker. In some embodiments, the linker comprises ((G)n(S))m, wherein n is an integer of 1-8, and m is an integer of 1-8. In some embodiments, the linker comprises 1-8 amino acids, such as Val-Cit and Glu-Val-Cit. In some embodiments, the linker comprises CO(O)C(CH2)ₙC(O)OH, wherein n is an integer of 2-8. In some embodiments, the linker comprises at least one of succinic acid, fumaric acid, glutaric acid, adipic acid, pimelic acid and suberic acid.

In some embodiments, the drug is selected from at least one of a small-molecule drug, an antibody, a polypeptide, a protein, a chemical drug, a chemotherapy drug and a fluorescent label. In some embodiments, the drug includes, but is not limited to, cytotoxic agents which can be any compound leading to cell death or inducing cell death, or reducing cell vitality in a certain manner. Exemplary cytotoxic agents comprise for example maytansine and maytansine analogs, taxanes (e.g., paclitaxel and docetaxel), CC-1065 and CC-1065 analogs, dolastatin and dolastatin analogs, methotrexate, daunorubicin, doxorubicin, vincristine, vinblastine, melphalan, mitomycin C, chlorambucil, calicheamicin, microtubulomycin and microtubulomycin analogs, and duocarmycin and duocarmycin analogs. In some preferred embodiments, the drug is selected from at least one of cytotoxic agents such as camptothecin, docetaxel and paclitaxel. The present application provides use of the peptide or derivative thereof, the drug-peptide conjugate or the pharmaceutical composition of the present disclosure in the preparation of a reagent or drug for detecting, preventing or treating a disease. The present application provides use of the peptide or derivative thereof, the polynucleotide, the expression vector, the host cell, the drug-peptide conjugate or the pharmaceutical composition of the present disclosure in the preparation of a reagent or drug for detecting, preventing or treating a disease.

In some embodiments, the disease comprises cancers. In some embodiments, the disease comprises solid tumors and/or hematological tumors. In some preferred embodiments, the disease comprises one or more of thyroid cancer, esophageal cancer, lung cancer, gastric cancer, liver cancer (e.g., highly metastatic liver cancer), cholangiocarcinoma, kidney cancer (e.g., renal cell adenocarcinoma and human clear cell renal cell carcinoma), pancreatic cancer, bowel cancer, colorectal cancer, bladder cancer, prostate cancer, breast cancer, uterine cancer, endometrial cancer, ovarian cancer, cervical cancer, fallopian tube cancer, skin cancer, brain tumor, glioma, melanoma, osteosarcoma, sarcoma, lymphoma, Hodgkin lymphoma, non-Hodgkin lymphoma, multiple myeloma or leukemia. In some embodiments, the tumor comprises one or more of non-small cell lung cancer, pulmonary giant cell carcinoma, gastric cancer, liver cancer, ovarian cancer, esophageal cancer, colon cancer, breast cancer, cholangiocarcinoma, osteosarcoma, cervical cancer, melanoma, bladder transitional cell carcinoma, pancreatic cancer, astrocytoma, chronic myeloid leukemia or lymphoma.

As used herein, the term "disease" refers to any change in the state of an organism or some organs that interrupt or disturb the performance of functions and/or causes symptoms (such as discomfort, dysfunction, adverse stress, or even death) in affected persons or persons in contact with the affected persons.

As used herein, the term "treating" refers to relieving or improving a disease or disorder (i.e., slowing down or preventing the development of the disease or at least one clinical condition); or relieving or improving at least one physical parameter or biological marker related to the disease or disorder.

The present application provides a method for detecting, preventing or treating a disease, the method comprising administrating to a subject in need thereof a detectably or preventively or therapeutically effective amount of the peptide or derivative thereof, the drug-peptide conjugate or the pharmaceutical composition of the present disclosure. The present application further provides a method for detecting, preventing or treating a disease, the method comprising administrating to a subject in need thereof a detectably or preventively or therapeutically effective amount of the peptide or derivative thereof, the polynucleotide, the expression vector, host cell, the drug-peptide conjugate or the pharmaceutical composition of the present disclosure.

In some embodiments, the disease comprises cancers. In some embodiments, the disease comprises solid tumors and/or hematological tumors. In some preferred embodiments, the disease comprises one or more of thyroid cancer, esophageal cancer, lung cancer, gastric cancer, liver cancer (e.g., highly metastatic liver cancer), cholangiocarcinoma, kidney cancer (e.g., renal cell adenocarcinoma and human clear cell renal cell carcinoma), pancreatic cancer, bowel cancer, colorectal cancer, bladder cancer, prostate cancer, breast cancer, uterine cancer, endometrial cancer, ovarian cancer, cervical cancer, fallopian tube cancer, skin cancer, brain tumor, glioma, melanoma, osteosarcoma, sarcoma, lymphoma, Hodgkin lymphoma, non-Hodgkin lymphoma, multiple myeloma or leukemia. In some embodiments, the tumor comprises one or more of non-small cell lung cancer, pulmonary giant cell carcinoma, gastric cancer, liver cancer, ovarian cancer, esophageal cancer, colon cancer, breast cancer, cholangiocarcinoma, osteosarcoma, cervical cancer, melanoma, bladder transitional cell carcinoma, pancreatic cancer, astrocytoma, acute lymphoblastic leukemia, chronic lymphoblastic leukemia, acute myeloid leukemia, chronic myeloid leukemia or lymphoma.

As used herein, the term "subject" comprises animals such as vertebrates, preferably mammals such as dogs, cats, pigs, cows, goats, horses, rodents (e.g., mice, rats or guinea pigs) or primates (e.g., gorilla, chimpanzee and human).

As used herein, the term "detectably or preventively or therapeutically effective dose" refers to an amount that results in a beneficial effect in detecting, preventing or treating a disease when compared to a corresponding subject who has not received such the amount, and which is sufficiently low within the scope of reasonable medical judgment to avoid serious side effects. The detectably or preventively or therapeutically effective dose of the peptide or derivative thereof, the polynucleotide, the expression vector, the host cell, the drug-peptide conjugate or the pharmaceutical composition described herein will vary with the selected peptide or derivative thereof, polynucleotide, expression vector, host cell, drug-peptide conjugate or pharmaceutical composition; administration routes; the severity of the disease; the age, bodily form, weight and physical condition of a patient: the patient's medical history the treatment duration; the nature of parallel treatment; and required detection; prevention or treatment effects and other factors, but is still determined by those skilled in the art in conventional manners.

Various embodiments for the peptide or derivative thereof, the polynucleotide, the expression vector and the host cell of the present disclosure are also suitable for the drug-peptide conjugate, the pharmaceutical composition, the use and the method (provided that they are not inherently contradictory to each other) of the present disclosure, and all various embodiments formed by such combination shall be deemed a part of the present disclosure.

### Examples

Next, exemplary embodiments of the present application will be described with reference to drawings, in which various details of the embodiments of the present application are included to help understanding. It should be understood that they are considered as being only exemplary, but are not intended to limit the protective scope of the present application. The protective scope of the present application is defined only by appended claims. Therefore, persons of ordinary skill in the art should recognize that various alternations and modifications can be made to the embodiments described herein without departing from the scope of the present application. Similarly, for clarity and conciseness, the descriptions of well-known functions and structures are omitted in the following description.

Unless otherwise specified, reagents and instruments used in the following examples are all conventional commercially available products. Unless otherwise specified, experiments are performed under conventional conditions or as recommended by manufacturers.

### Example 1: Preparation of peptide

### 1. Preparation of peptide

1 g of Wang resin (CAS:1365700-43-1) with a degree of substitution of 1.1 mmol/g was weighed and added into a polypeptide solid-phase reactor (Glass instruments, Shanghao Jikun Chemical Technology Co., Ltd.). Dichloromethane (DCM) (Energy Chemical) was added into the above reactor, and the resin was swelled with continuous nitrogen bubbling for 10-20 min. Then, the solvent in the reactor was drained. 0.345 g of Fmoc-S(tBu)-OH (Sichuan Zhengyuan Biotechnology Co., Ltd.), 130 mg of dimethylaminopyridine (DMAP) and 0.405 g of hydroxybenzotriazole (HOBT) were accurately weighed and dissolved in 5-10 ml of dimethyl formamide (DMF) as a solvent, 2 ml of diisopropylcarbodiimide (DIC) was added (all the above reagents were purchased from Energy Chemical unless otherwise specified), and then the above reaction system reacted at room temperature with nitrogen sparging for 4-8 h, and the reaction mixture was drained, and then washed twice with DMF.

The resin was capped with acetic anhydride. Specifically, 5 ml of DCM was used as the solvent, and 2 ml of N,N-diisopropylethylamine (DIPEA) and 2 ml of acetic anhydride were additionally added into the reaction system (all the reagents were purchased from Energy Chemical); the above reaction system reacted at room temperature with nitrogen sparging for 20-30 min; and after the reaction was ended, the resin was washed 4 times with DMF.

Fmoc was removed twice using a commercially available 20% piperidine/DMF solution (100 ml/400 ml). The reaction system reacted at room temperature with nitrogen sparging for 10 min and 5 min respectively. After the reaction was ended, the resin was washed 5 times with DMF, and then detected for 30-60 s using commercially available ninhydrin: phenol:piperidine (2:1:1). If the resin turned dark blue, Fmoc had been successfully removed.

1.612 g of Fmoc-N(trt)-OH (Sichuan Zhengyuan Biotechnology Co., Ltd.) and 1.023 g of commercially available O-benzotriazole-N,N,N',N'-tetramethyluronium hexafluorophosphate (HBTU) were weighed and added into the polypeptide solid-phase reactor, and 5 ml of DMF serving as the solvent and 1 ml of N,N-diisopropylethylamine (DIPEA) were added into the reactor. The above reaction system reacted at room temperature with nitrogen sparging for 40-60 min. The solvent in the reactor was drained. The resin was washed 4 times with DMF and then detected for 2-3 min using ninhydrin: phenol:piperidine (2:1:1). If the resin was colorless, coupling was successful.

Fmoc was removed twice using a 20% piperidine/DMF solution (100 ml/400 ml). The reaction system reacted at room temperature with nitrogen sparging for 10 min and 5 min respectively. After the reaction was ended, the resin was washed 5 times with DMF, and then detected for 30-60 s using commercially available ninhydrin: phenol:piperidine (2:1:1). If the resin turned dark blue, Fmoc had been successfully removed.

The above operations were repeated, and each amino acid was successively added in the peptide sequence. According to the peptide sequence, the resin was coupled with Fmoc-A-OH, Fmoc-Y(tBu)-OH, Fmoc-P-OH, Fmoc-S(tBu)-OH and Fmoc-R(pbf)-OH (the above reagents were all purchased from Sichuan Zhengyuan Biotechnology Co., Ltd.) in sequence, and then a Fmoc protecting group was removed using a 20% piperidine/DMF solution (100 ml/400 ml).The solution was washed 6 times with DMF. The resin was shrunk twice with methanol. The residual liquid in the resin was drained to obtain a P1-AI protected peptide resin.

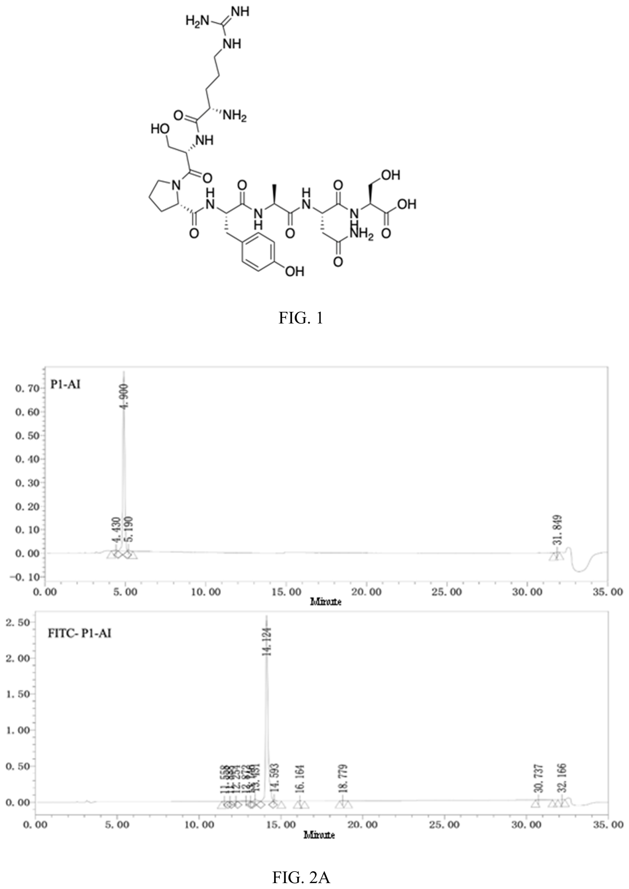

15.2 ml of commercially available trifluoroacetic acid (TFA), 0.8 ml of H₂O and 0.8 ml of commercially available Tis were used as lysate. The P1-AI protected peptide resin and the lysate were jointly placed in a centrifugal tube and reacted for 1.5-2 h. After that, the reaction product was subjected to sedimentation with ice diethyl ether and centrifuged three times to obtain a white solid. The obtained white solid was dried in a vacuum dry oven to obtain a crude product with the purity of 90%. The obtained crude product was purified via HPLC and lyophilized to obtain a white powder peptide P1-AI (SEQ ID NO:1). The structure of P1-AI is as shown in FIG. 1.

### 2. Preparation of FITC-P1-AI peptide

0.1 g of Wang resin with a degree of substitution of 1.1 mmol/g was weighed and added into a peptide solid-phase reactor. 5 ml of DCM was added into the above reactor, and the resin was swelled for 10 min with nitrogen bubbling, and then the solvent in the reactor was drained. 0.0348 g of Fmoc-S(tBu)-OH, 13 mg of DMAP and 0.0408 g of HOBT were accurately weighed and dissolved in DMF serving a solvent, 0.2 ml of DIC was added, and then the above reaction system reacted at room temperature with nitrogen sparging for 4-8 h, and the reaction mixture was drained, and then washed twice with DMF.

The resin was capped with acetic anhydride. Specifically, 5 ml of DCM was used as the solvent, and 0.2 ml of DIPEA and 0.2 ml of acetic anhydride were added into the reaction system; the above reaction system reacted at room temperature with nitrogen sparging for 20-30 min; and after the reaction was ended, the resin was washed 4 times with DMF.

Fmoc was removed twice using a 20% piperidine/DMF solution. The reaction system reacted at room temperature with nitrogen sparging for 10 min and 5 min respectively. After the reaction was ended, the resin was washed 5 times with DMF, and then detected for 30-60 s using ninhydrin: phenol:piperidine (2:1:1). If the resin turned dark blue, Fmoc had been successfully removed.

0.1161 g of Fmoc-N(trt)-OH and 0.1023 g of HBTU were weighed and added into the reactor, and DMF serving as the solvent and 0.1 ml of DIPEA were added into the reactor. The above reaction system reacted at room temperature with nitrogen sparging for 60 min. The solvent in the reactor was drained. The resin was washed 4 times with DMF and then detected for 2-3 min using ninhydrin: phenol:piperidine (2:1:1). If the resin was colorless, coupling was successful.

Fmoc was removed twice using a 20% piperidine/DMF solution. The reaction system reacted at room temperature with nitrogen sparging for 10 min and 5 min respectively. After the reaction was ended, the resin was washed 5 times with DMF, and then detected for 30-60 s using ninhydrin: phenol:piperidine (2:1:1). If the resin turned dark blue, Fmoc had been successfully removed.

The above operations were repeated, and each amino acid was successively added in the peptide sequence. According to the peptide sequence, the resin was coupled with Fmoc-A-OH, Fmoc-Y(tBu)-OH, Fmoc-P-OH, Fmoc-S(tBu)-OH and Fmoc-R(pbf)-OH in sequence, and then a Fmoc protecting group was removed using a 20% piperidine/DMF solution.

0.381 g of Fmoc-Acp-OH and 0.1023 g of HBTU were weighed and added into the reactor. DMF serving as the solvent and 0.1 ml of DIPEA were added into the reactor. The above reaction system reacted at room temperature with nitrogen sparging for 3 h. The solvent in the reactor was drained. The resin was washed 4 times using DMF and then detected for 2-3 min using ninhydrin: phenol:piperidine (2:1:1). If the resin was colorless, coupling was successful.

Fmoc was removed twice using a 20% piperidine/DMF solution with reaction lasting 10 min and 5 min respectively. After the reaction was ended, the resin was washed 5 times with DMF, and then detected for 30-60 s using ninhydrin: phenol:piperidine (2:1:1). If the resin turned dark blue, Fmoc had been successfully removed.

0.14 g of FITC was weighed and added into the reactor, and then DMF serving as the solvent and 1 ml of triethylamine were added into the above reactor. The above reaction system reacted for 4 h in the dark, and then the solution was washed 6 times with DMF. The resin was shrunk twice with methanol. The residual liquid in the resin was drained to obtain a FITC-P1-AI protected peptide resin.

7.6 ml of TFA, 0.4 ml of H₂O and 0.4 ml of Tis were used as lysate. The FITC-P1-AI protected peptide resin and the lysate were jointly placed in a centrifugal tube and reacted for 1.5-2 h. After that, the reaction product was subjected to sedimentation with ice diethyl ether and centrifuged three times to obtain a yellow solid. The obtained yellow solid was dried in a vacuum dry oven to obtain a crude product with the purity of 90%. The obtained crude product was purified via HPLC and lyophilized to obtain yellow powder peptide FITC-P1-AI.

### 3. Preparation of Cy5-P1-AI peptide

0.1 g of Wang resin with a degree of substitution of 1.1 mmol/g was weighed and added into a peptide solid-phase reactor. 5 ml of DCM was added into the above reactor. The resin was swelled for 10 min with nitrogen bubbling. The solvent in the reactor was drained. 0.0348 g of Fmoc-S(tBu)-OH, 13 mg of DMAP and 0.0408 g of HOBT were accurately weighed and dissolved in DMF serving as a solvent, 0.2 ml of DIC was added into the above reactor, and then the above reaction system reacted at room temperature with nitrogen sparging for 4-8 h, and then the reaction mixture was drained, and then washed twice with DMF.

The resin was capped with acetic anhydride. Specifically, DCM was used as the solvent, and 0.2 ml of DIPEA and 0.2 ml of acetic anhydride were added into the reaction system. The above reaction system reacted at room temperature with nitrogen sparging for 20-30 min; and after the reaction was ended, the resin was washed 4 times with DMF.

Fmoc was removed twice using a 20% piperidine/DMF solution. The reaction system reacted at room temperature with nitrogen sparging for 10 min and 5 min respectively. After the reaction was ended, the resin was washed 5 times with DMF, and then detected for 30-60 s using ninhydrin: phenol:piperidine (2:1:1). If the resin turned dark blue, Fmoc had been successfully removed.

0.1161 g of Fmoc-N(trt)-OH and 0.1023 g of HBTU were weighed and added into the reactor, DMF serving as the solvent and 0.1 ml of DIPEA were added into the reactor. The above reaction system reacted at room temperature with nitrogen sparging for 60 min. The solvent in the reactor was drained. The resin was washed 4 times with DMF and then detected for 2-3 min using ninhydrin: phenol:piperidine (2:1:1). If the resin was colorless, coupling was successful.

Fmoc was removed twice using a 20% piperidine/DMF solution. The reaction system reacted at room temperature with nitrogen sparging for 10 min and 5 min respectively. After the reaction was ended, the resin was washed 5 times with DMF, and then detected for 20-30 s using ninhydrin: phenol:piperidine (2:1:1). If the resin turned dark blue, Fmoc had been successfully removed.

The above operations were repeated, and each amino acid was successively added in the peptide sequence. According to the peptide sequence, the resin was coupled with Fmoc-A-OH, Fmoc-Y(tBu)-OH, Fmoc-P-OH, Fmoc-S(tBu)-OH and Fmoc-R(pbf)-OH in sequence, and then a Fmoc protecting group was removed using a 20% piperidine/DMF solution. The solution was washed 6 times with DMF. The resin was shrunk twice with methanol. The residual liquid in the resin was drained to obtain a P1-AI protected peptide resin.

100 mg of sulfo-Cy5 carboxylic acid, excessive P1-AI protected peptide resin and 0.1023 g of HBTU were weighed and added into the reactor, and DMF serving as the solvent and 0.1 ml of DIPEA were added into the reactor. The above reaction system reacted for 2 h in the dark, and then the solution was washed 6 times using DMF. The resin was shrunk twice with methanol, and then the residual liquid in the resin was drained to obtain a Cy5-P1-AI protected peptide resin.

7.6 ml of TFA, 0.4 ml of H₂O and 0.4 ml of Tis were used as lysate. The Cy5-P1-AI protected peptide resin and the lysate were jointly placed in a centrifugal tube and reacted for 1.5-2 h. After that, then the reaction product was subjected to sedimentation with ice diethyl ether and centrifuged three times to obtain a blue solid. The obtained blue solid was dried in a vacuum dry oven to obtain a crude product with the purity of 90%. The obtained crude product was purified via HPLC and lyophilized to obtain blue powder peptide Cy5-P1-AI.

### 4. Preparation of other peptides

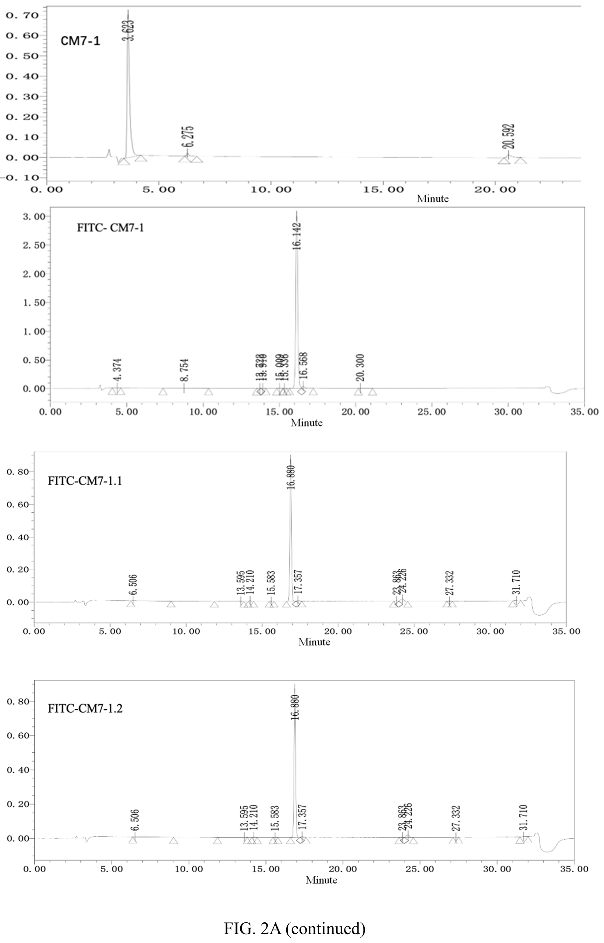

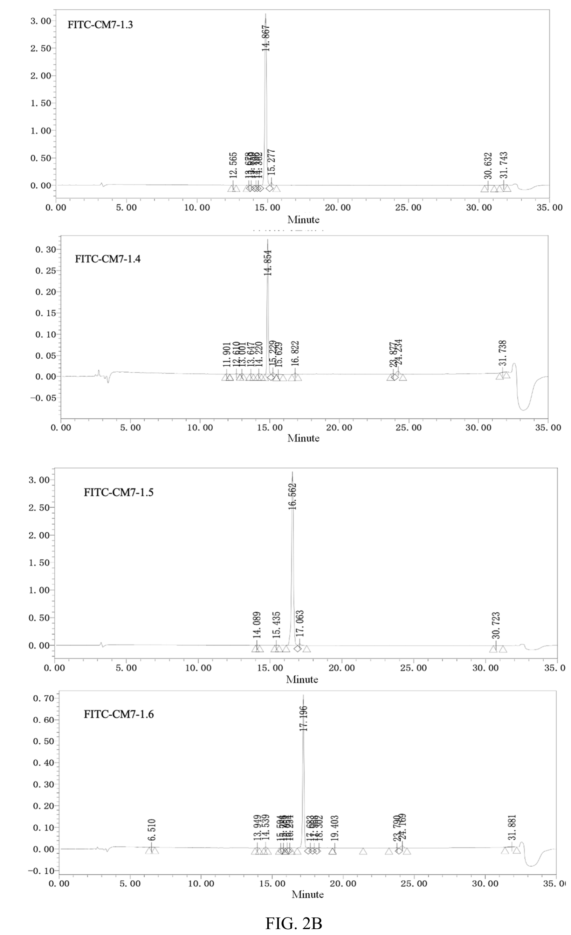

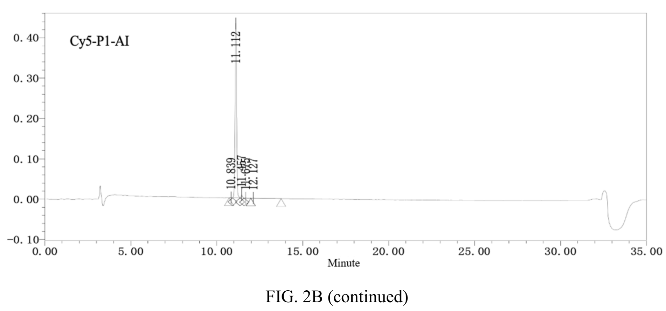

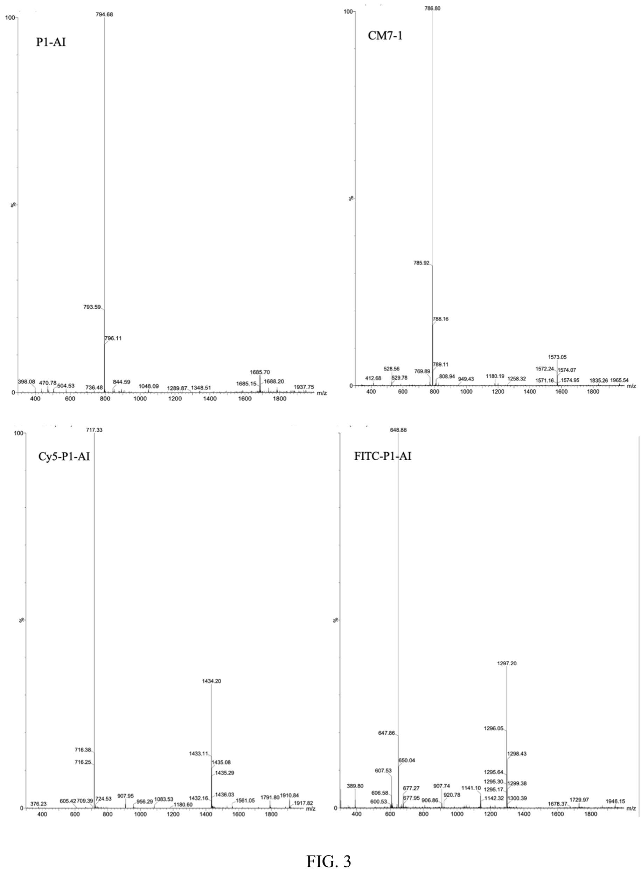

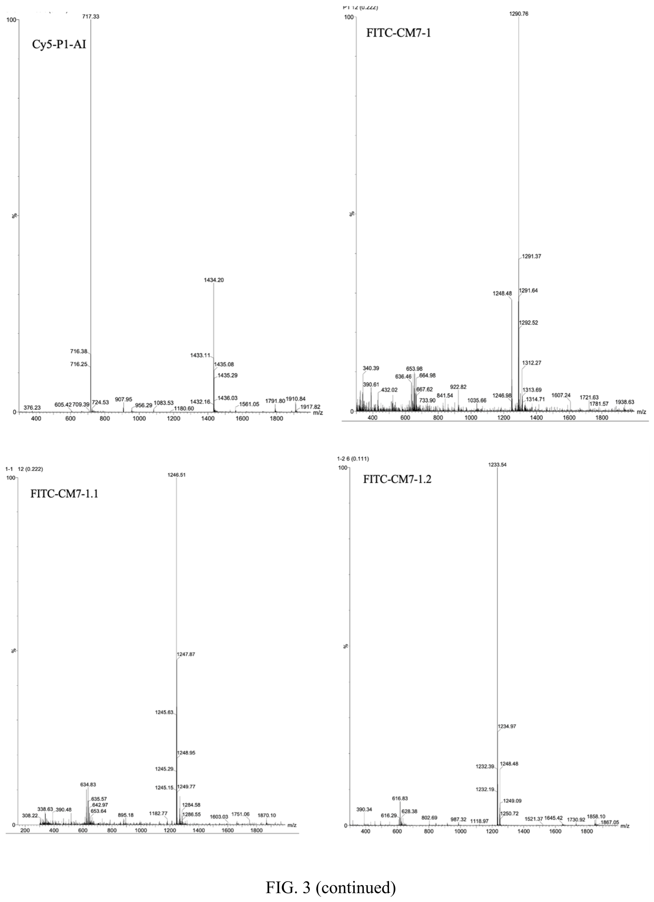

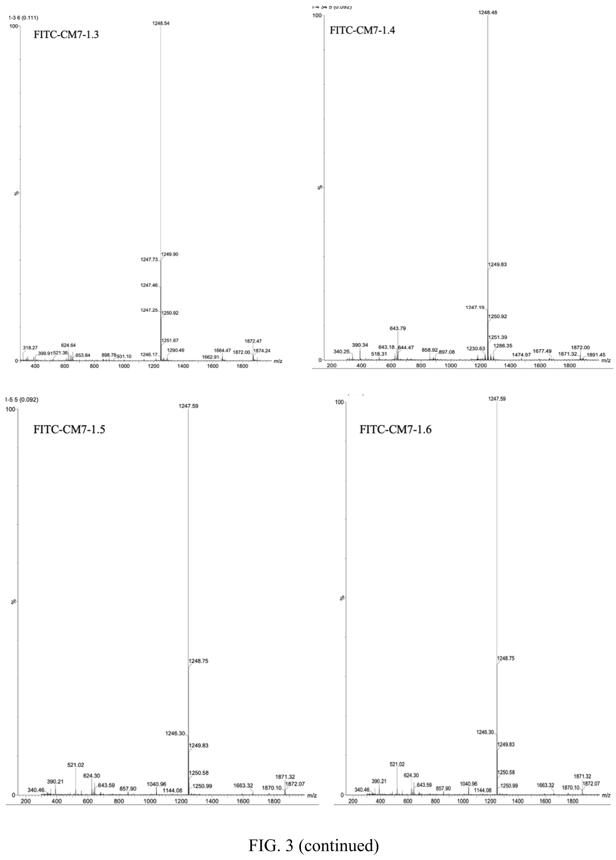

By using the above solid-phase synthesis method, peptides P1-AI (RSPYANS, with a molecular weight of 793.82), FITC-P1-AI (FITC-RSPYANS, with a molecular weight of 1296.36), Cy5-P1-AI (Cy5-RSPYANS, with a molecular weight of 1432.63), CM7-1 (DQIIANN, with a molecular weight of 786.83, corresponding to an optimal polypeptide designed in CN 112979753 B), FITC-CM7-1 (FITC-DQIIANN, with a molecular weight of 1289.36), FITC-CM7-1.1 (FITC-AQIIANN, with a molecular weight of 1245.35), FITC-CM7-1.2 (FITC-DAIIANN, with a molecular weight of 1232.31), FITC-CM7-1.3 (FITC-DQAIANN, with a molecular weight of 1247.28), FITC-CM7-1.4 (FITC-DQIAANN, with a molecular weight of 1247.28), FITC-CM7-1.5 (FITC-DQIIAAN, with a molecular weight of 1246.34) and FITC-CM7-1.6 (FITC-DQIIANA, with a molecular weight of 1246.34) were obtained. The peptides involved here and sequences thereof are listed in Table 1. The HPLC detection results are as shown in FIG. 2A and FIG. 2B, and the mass spectrometry analysis results are as shown in FIG. 3.

**Table 1 Peptides and Their Sequences in Example 1**

| SEQ ID NO | Name | Amino Acid Sequence |
|---|---|---|
| 1 | P1-AI | RSPYANS |
| 2 | CM7-1 | DQIIANN |
| 3 | CM7-1.1 | AQIIANN |
| 4 | CM7-1.2 | DAIIANN |
| 5 | CM7-1.3 | DQAIANN |
| 6 | CM7-1.4 | DQIAANN |
| 7 | CM7-1.5 | DQIIAAN |
| 8 | CM7-1.6 | DQIIANA |

### Example 2: The detection of c-Met expression in different cells via flow cytometry

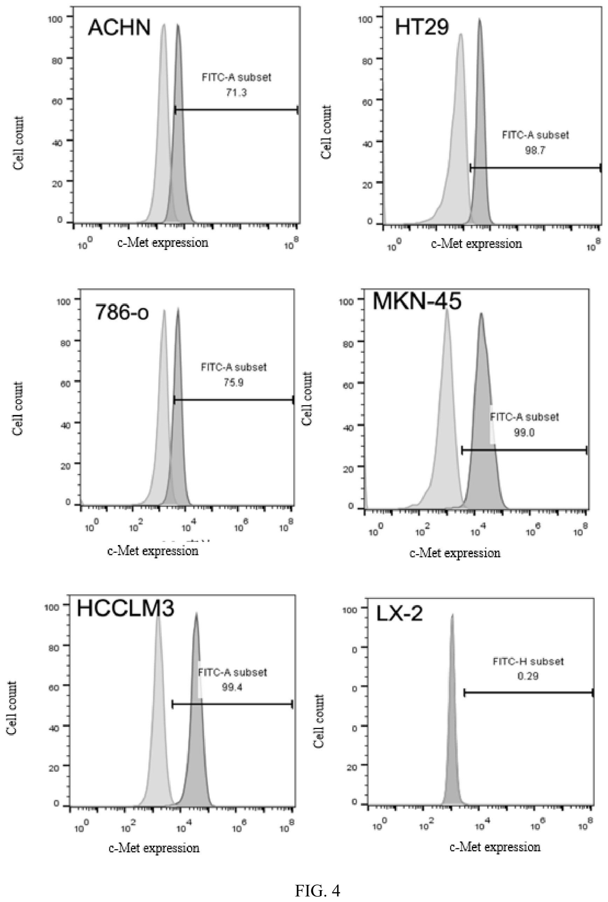

Human gastric cancer cell MKN-45 (Beijing Beina Chuanglian Biotechnology Research Institute), highly metastatic human hepatocellular carcinoma cell HCCLM3, human colon cancer cell HT29 (Beijing Beina Chuanglian Biotechnology Research Institute), renal cell adenocarcinoma cell ACHN, human clear cell renal cell carcinoma cell 786-O and human hepatic stellate cell LX-2 (all the above cells were purchased from Nanjing Saihongrui Biotechnology Co., Ltd. unless otherwise specified) were revived, cultured and passaged more than three times for later use; and after three-generation cell culture, the cells were washed several times with pre-cooled commercially available PBS (4°C) under proper density conditions to remove poorly viable cells and floating debris, followed by digestion with a trypsin solution; after the cells rounded up, an equal volume of 1640 or DMEM culture medium was added to terminate the digestion, and then the digested product was centrifuged at 1000 rpm for 5 min; supernatant was discarded, and the cells were blocked with a 2% commercially available bovine serum albumin (BSA) blocking solution at 4°C for 1 h; 100 µl of cells with the above concentration were respectively transferred to a 1.5 ml centrifuge tube, and an antihuman FITC-c-Met antibody (purchased from eBioscience) or FITC-c-Met isotype control IgG (purchased from eBioscience) was added respectively into the centrifuge tube, and then the cells were incubated in the dark at 4°C for 30 min; and the above cells were then centrifuged at 2000 rpm for 5 min at 4°C, washed 3 times with PBS, and detected using a flow cytometer (CytoFLEX). The results are as shown in FIG. 4. FIG. 4 shows the flow cytometry detection results of c-Met expression in different cells, wherein the horizontal axis represents a fluorescence signal, and the longitudinal axis represents the quantity of cells.

The results show that HCCLM3, HT29, MKN-45, ACHN and 786-0 significantly and highly expressed the c-Met protein, while the human hepatic stellate cell LX-2 barely expressed the c-Met protein.

### Example 3: Affinity detection of peptide and c-Met positive cell MKN45

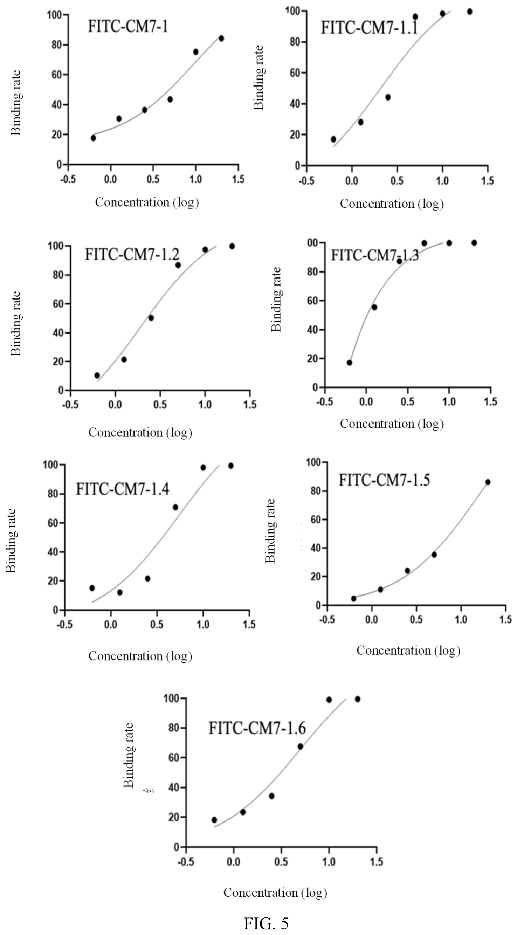

MKN 45 cells cultured in a 1640 culture solution (Nanjing Senbeijia Biotechnology Co., Ltd.) were collected, and washed twice with pre-cooled PBS. 1 mL of 1% BSA solution was added into the washed cells, and then the above mixed system was placed on a rotary mixer (Lichen Technology Co., Ltd.) and incubated for 30 min at 4°C. Different concentrations of FITC-P1-AI, FITC-CM7-1, FITC-CM7-1.1, FITC-CM7-1.2, FITC-CM7-1.3, FITC-CM7-1.4, FITC-CM7-1.5 and FITC-CM7-1.6 peptide solutions were respectively added in the dark to obtain multiple groups, and then the mixtures were placed on the rotary mixer and incubated for 1 h at 4°C in the dark. After the incubation was completed, the mixed system of the cells was centrifuged for 5 min at 800 rpm, and then supernatant was discarded. The cells were washed twice with pre-cooled PBS, and the concentration of the cells was adjusted to 1×10⁶ cells/mL using PBS. 3 parallel replicate wells were set up for cells in each group, with 0.5 mL prepared per sample; and a flow cytometer was used to detect the binding of the peptide to c-Met positive cell MKN-45, EC50 data and a binding curve were generated. The results are as shown in Table 2 and FIG. 5. FIG. 5 shows the binding curve of the peptide of the present disclosure and the c-Met positive cell MKN-45, wherein the horizontal axis represents log (polypeptide concentration), and the unit is µM; and the longitudinal axis represents a binding rate (%).

**Table 2 Binding affinity of peptide to MKN-45**

| Name | EC50(*µ*M) |
|---|---|
| FITC-CM7-1 | 9.505 |
| FITC-CM7-1.1 | 2.073 |
| FITC-CM7-1.2 | 1.884 |
| FITC-CM7-1.3 | 0.0456 |
| FITC-CM7-1.4 | 5.367 |
| FITC-CM7-1.5 | 15.22 |
| FITC-CM7-1.6 | 5.053 |

The results show that the EC50 of CM7-1.1, CM 7-1.2 and CM 7-1.3 as well as CM7-1.4 and CM7-1.6 is lower than that of CM 7-1 (an optimal polypeptide designed in CN 112979753 B), indicating that CM7-1.1, CM 7-1.2, CM 7-1.3, CM7-1.4 and CM7-1.6 have an increased affinity to c-Met positive cell MKN45 relative to CM7-1. Specifically, the EC50 of CM7-1.2 and CM7-1.3 is significantly lower than that of CM 7-1, indicating that CM7-1.2 and CM7-1.3 have significantly increased affinity to c-Met positive cell MKN45 relative to CM7-1. In addition, the binding of CM7-1.2 and CM7-1.3 to c-Met positive cell MKN45 is dose-dependent.

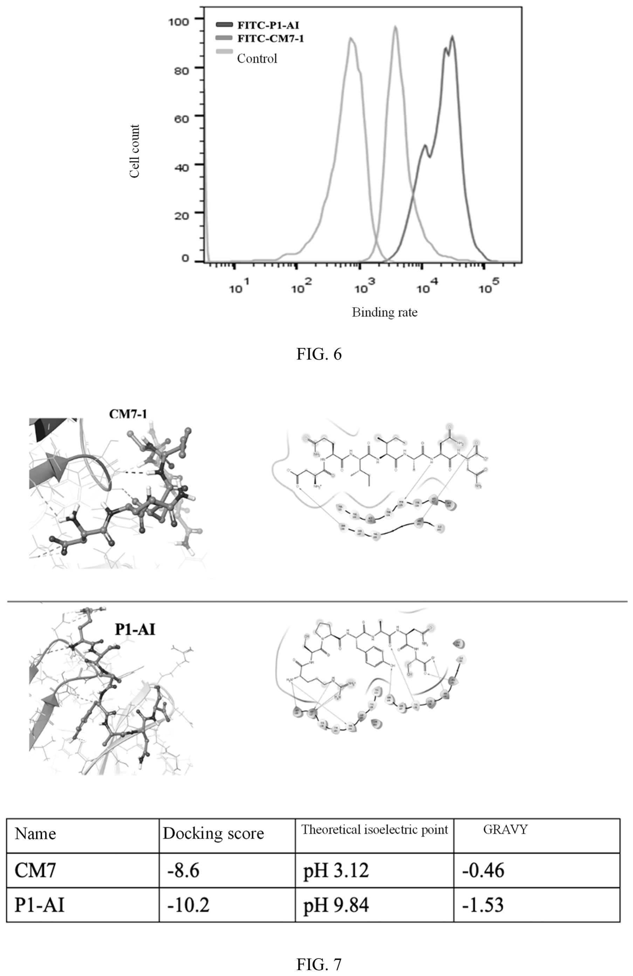

MKN-45 cells cultured in a 1640 culture solution were collected, and washed twice with pre-cooled PBS 1 mL of 1% BSA solution was added into the washed cells, and then the above mixed system was placed on a rotary mixer and incubated for 30-60 min at 4°C. 10 µM of FITC-P1-AI peptide solution and FITC-CM7-1 were respectively added in the dark to form two groups, and the above mixed systems were placed on the rotary mixer and incubated for 1 h at 4°C in the dark. After the incubation was completed, the mixed system of the cells was centrifuged for 5 min at 800 rpm, and then supernatant was discarded. The cells were washed 1-2 times with pre-cooled PBS, and then the concentration of the washed cells was adjusted to 1×10⁶ cells/mL by using PBS. 3 parallel replicate wells were set up for cells in each group, with 0.5 mL prepared per sample; and a flow cytometer was used to detect the binding of the peptide to c-Met positive cells. The results are shown in FIG. 6. FIG. 6 shows the binding curve of the peptide of the present disclosure and c-Met positive cell MKN-45, wherein the horizontal axis represents a fluorescence signal, and the longitudinal axis represents the quantity of cells.

The results show the binding of P1-AI and CM7-1 to c-Met positive cell MKN45. Further, compared with CM7-1, the P1-AI peptide exhibits a significantly increased affinity to c-Met positive cell MKN45.

### Example 4: Molecule docking

The amino acid at each position of a CM7-1 peptide sequence was replaced and docked by using a computer and alanine scanning, and the amino acid sequences after replacement, as shown in Table 3, were obtained. The docking scores of these peptide sequences and c-Met are also presented in Table 3.

**Table 3 Molecule Docking Analysis**

| SEQ ID NO. | Sequence | Docking Score | SEQ ID NO. | Sequence | Docking Score |
|---|---|---|---|---|---|
| 9 | HQIIANN | -9.3 | 78 | RSPNANN | -6.7 |
| 10 | RQIIANN | -10 | 79 | RSPSANN | -8.7 |
| 11 | FQIIANN | -9.9 | 80 | RSPYANN | -9.9 |
| 12 | AQIIANN | -9.8 | 81 | RSPTANN | -8.7 |
| 13 | CQIIANN | -9.4 | 82 | RSPWANN | -7.5 |
| 14 | GQIIANN | -9.8 | 83 | RSPPANN | -8.5 |
| 15 | QQIIANN | -8.9 | 84 | RSPVANN | -9.7 |
| 16 | EQIIANN | -8.8 | 85 | RSPYANN | -9.9 |
| 17 | KQIIANN | -9.6 | 86 | RSPYHNN | -8.3 |
| 18 | LQIIANN | -8.7 | 87 | RSPYDNN | -0.3 |
| 19 | MQIIANN | -8.6 | 88 | RSPYRNN | -1.5 |
| 20 | NQIIANN | -9.2 | 89 | RSPYFNN | -7.3 |
| 21 | SQIIANN | -8 | 90 | RSPYCNN | -10.3 |
| 22 | YQIIANN | -8.5 | 91 | RSPYGNN | -6.4 |
| 23 | TQIIANN | -7.6 | 92 | RSPYQNN | -6.2 |
| 24 | IQIIANN | -7.4 | 93 | RSPYENN | -6.7 |
| 25 | WQIIANN | -8.2 | 94 | RSPYKNN | -5.4 |
| 26 | PQIIANN | -8.1 | 95 | RSPYLNN | -7.6 |
| 27 | VQIIANN | -7 | 96 | RSPYMNN | -9 |
| 28 | RHIIANN | -9.5 | 97 | RSPYNNN | -4.2 |
| 29 | RDIIANN | -4.2 | 98 | RSPYSNN | -6.8 |
| 30 | RRIIANN | -5.1 | 99 | RSPYYNN | 4.9 |
| 31 | RFIIANN | -4.6 | 100 | RSPYTNN | -7.6 |
| 32 | RAIIANN | -6.4 | 101 | RSPYINN | -1.4 |
| 33 | RCIIANN | -6.3 | 102 | RSPYWNN | -4.1 |
| 34 | RGIIANN | -6.9 | 103 | RSPYPNN | -7.7 |
| 35 | REIIANN | -9.2 | 104 | RSPYVNN | -8 |
| 36 | RKIIANN | -4.4 | 105 | RSPYANN | -9.9 |
| 37 | RLIIANN | -8 | 106 | RSPYAHN | -6.5 |
| 38 | RMIIANN | -6.4 | 107 | RSPYADN | -7.3 |
| 39 | RNIIANN | -7 | 108 | RSPYARN | -7.1 |
| 40 | RSIIANN | -9.6 | 109 | RSPYAFN | -7.2 |
| 41 | RYIIANN | -6.4 | 110 | RSPYAAN | -4.7 |
| 42 | RTIIANN | -3.2 | 111 | RSPYACN | -9.7 |
| 43 | RIIIANN | -4.9 | 112 | RSPYAGN | -6.5 |
| 44 | RWIIANN | -4.2 | 113 | RSPYAQN | -6.6 |
| 45 | RPIIANN | -7.8 | 114 | RSPYAEN | -6.4 |
| 46 | RVIIANN | -8.1 | 115 | RSPYAKN | -5.8 |
| 47 | RSHIANN | -4.6 | 116 | RSPYALN | 0.5 |
| 48 | RSDIANN | -8.3 | 117 | RSPYAMN | -8.2 |
| 49 | RSRIANN | -5.9 | 118 | RSPYASN | -7.3 |
| 50 | RSFIANN | -6.5 | 119 | RSPYAYN | -6.5 |
| 51 | RSAIANN | -7.6 | 120 | RSPYATN | -8.3 |
| 52 | RSCIANN | -8 | 121 | RSPYAIN | -6.4 |
| 53 | RSGIANN | -6.4 | 122 | RSPYAWN | -4.8 |
| 54 | RSQIANN | -7.8 | 123 | RSPYAPN | -9.5 |
| 55 | RSEIANN | -4.2 | 124 | RSPYAVN | -6.7 |
| 56 | RSKIANN | -7.4 | 125 | RSPYANN | -9.2 |
| 57 | RSLIANN | -5.5 | 126 | RSPYANH | -7.1 |
| 58 | RSMIANN | -7.4 | 127 | RSPYAND | -8.3 |
| 59 | RSNIANN | -7.7 | 128 | RSPYANR | -1.7 |
| 60 | RSSIANN | -6.9 | 129 | RSPYANF | -6.2 |
| 61 | RSYIANN | -5.4 | 130 | RSPYANA | -8.1 |
| 62 | RSTIANN | -7.8 | 131 | RSPYANC | -6.7 |
| 63 | RSWIANN | -4.1 | 132 | RSPYANG | -5.5 |
| 64 | RSPIANN | -9.7 | 133 | RSPYANQ | -5.5 |
| 65 | RSVIANN | -7.6 | 134 | RSPYANE | -8.1 |
| 66 | RSPHANN | -9.2 | 135 | RSPYANK | -8.6 |
| 67 | RSPDANN | -7.6 | 136 | RSPYANL | -5.3 |
| 68 | RSPRANN | -7.3 | 137 | RSPYANM | -6.1 |
| 69 | RSPFANN | -3.1 | 1 (P1-AI) | RSPYANS | -10.2 |
| 70 | RSPAANN | -8.5 | 138 | RSPYANY | -6.9 |
| 71 | RSPCANN | -8.2 | 139 | RSPYANT | -5.5 |
| 72 | RSPGANN | -9.3 | 140 | RSPYANI | -7.8 |
| 73 | RSPQANN | -7.8 | 141 | RSPYANW | -5.6 |
| 74 | RSPEANN | -6.3 | 142 | RSPYANP | -4.7 |
| 75 | RSPKANN | -7.3 | 143 | RSPYANV | -5.9 |
| 76 | RSPLANN | -6.9 | 2 (CM7-1) | DQIIANN | -8.6 |
| 77 | RSPMANN | -2.1 | 144 (P1-AC) | RSPYCNS | -10.47 |

Where, polypeptides P1-AI: Arg-Ser-Pro-Tyr-Ala-Asn-Ser and CM7-1: Asp-Gln-Ile-Ile-Ala-Asn-Asn were respectively subjected to molecular docking with the c-Met protein. As shown in FIG. 7, the docking score of P1-AI is -10.162, the grand average of hydrophilicity (GRAVY) (a negative value represents hydrophilicity, the smaller the GRAVY value is, the stronger the hydrophilicity is) is -0.46; and the docking score of CM7-1 is -3.724, and GRAVY is -1.53.Therefore, the results show that the docking score of P1-AI is far superior to that of CM7-1, and the theoretical water solubility of P1-A is better than that of CM7-1.

### Example 5: Specific binding detection of peptide to c-Met protein

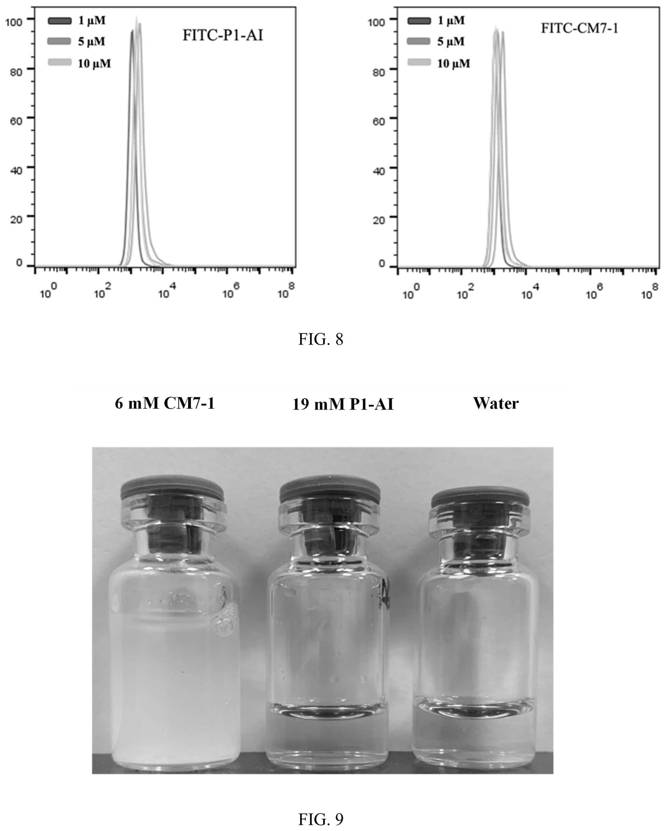

Human hepatic stellate cells LX-2 cultured in a 1640 culture solution were collected, and washed twice with pre-cooled PBS. 1 mL of 1% BSA solution was added into the washed cells, and then the above mixed system were placed on a rotary mixer and incubated for 30 min at 4°C. 10 µM of FITC-P1-AI peptide solution and FITC-CM7-1 were respectively added in the dark to form two groups, and the above mixed systems were placed on the rotary mixer and incubated for 1 h at 4°C in the dark. After the incubation was completed, the mixed system of the cells was centrifuged for 5 min at 800 rpm, and then supernatant was discarded. The cells were washed twice with pre-cooled PBS, and the concentration of the cells was adjusted to 1×10⁶ cells/mL using PBS. 3 parallel replicate wells were set up at concentrations of 1 µM, 5 µM and 10 µM for cells in each group, with 0.5 mL prepared per sample; and a flow cytometer was used to detect the binding of the peptide to LX-2 cells. The results are shown in FIG. 8. FIG. 8 shows the binding curve of the peptide of the present disclosure and human hepatic stellate cell LX-2 that barely expresses c-Met, wherein the horizontal axis represents a fluorescence signal, and the longitudinal axis represents the quantity of cells.

It is proved in example 2 that the human hepatic stellate cell LX-2 barely expresses c-Met. The results in FIG. 8 show that at the concentration of up to 10 µM, neither FITC-P1-AI nor FITC-CM7-1 barely binds to LX-2 cells. Comprehensively considering the results presented in FIG. 6-FIG. 8, both P1-AI and CM7-1 peptides can specifically bind to the c-Met protein, and the binding performance of P1-AI is superior to that of CM7-1 as described above.

### Example 6: Solubility detection of peptide

13.4 mg of CM7-1 and 15 mg of P1-AI were respectively weighed and dissolved using ultrapure (UP) water (prepared using Youpu Pure Water/Ultrapure Water Manufacturing System (UPR-II-10T)). The results are as shown in FIG. 9. FIG. 9 shows the water solubility detection results of the peptide of the present disclosure.

The results show that when the concentration was 6 mM, CM7-1 presented a completely cloudy state in water; and when the concentration was 19 mM, P1-AI formed a clear solution in water, it was observed by nude eyes that its state was the same as that of water. It indicates that the water solubility of peptide P1-AI is far superior to that of CM7-1, which is more beneficial for subsequent drug development and preparation.

### Example 7: The detection of interaction between peptide and c-Met protein by using microscale thermophoresis (MST)

FITC-P1-AI and FITC-CM7-1 were prepared into different concentrations of liquids, peptide solutions were subjected to siphon using a capillary, and a fluorescence absorption value was measured by using an MST instrument (OCTED RED 96e).

The c-Met protein was serially diluted 2-fold using PBS to form 12 concentration gradients with the concentrations of 10 µM, 5 µM, 2.5 µM, 1.25 µM, 0.625 µM, 0.32 µM, 0.16 µM, 0.08 µM, 0.04 µM, 0.02 µM, 0.01 µM and 0.005 µM, respectively. The c-Met protein diluted solution was mixed with FITC-P1-AI or FITC-CM7-1 in a volume ratio of 1:1, and the obtained mixture was subjected to standing.

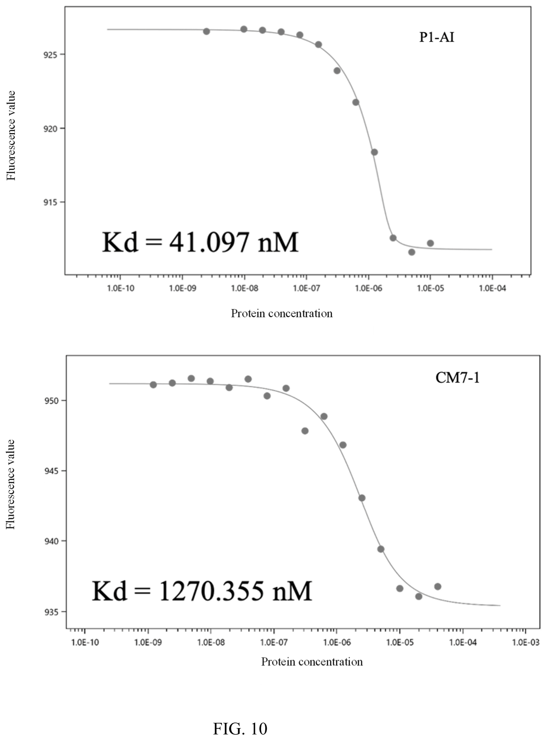

The affinity of FITC-P1-AI or FITC-CM7-1 to the c-Met protein was calculated. Data fitting was performed using NT Analysis software. The results are as shown in FIG. 10. FIG. 10 shows the results of interaction between the peptide of the present disclosure and the c-Met protein, wherein the horizontal axis represents the concentration of the c-Met protein, and the unit is µM; and the longitudinal axis represents fluorescence intensity.

The results show the affinity of P1-AI to the c-Met protein was Kd=41.097 nM, while the affinity of CM7-1 to the c-Met protein was only Kd=1270.355 nM. It can be seen that compared with CM7-1, the affinity of P1-AI to the c-Met protein was increased by 30.9 fold.

### Example 8: Living imaging study of transplant subcutaneous sarcoma of nude mice

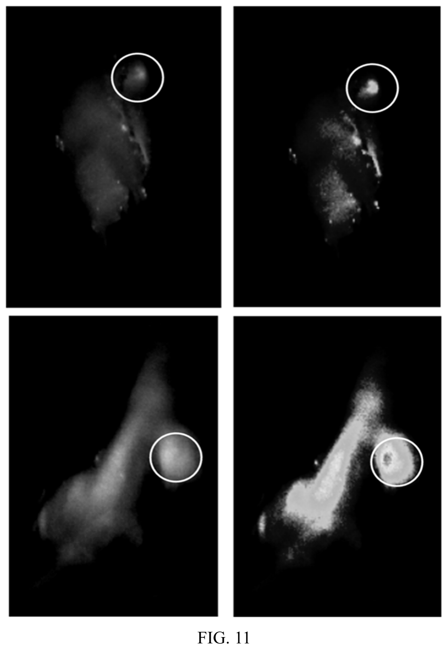

In MKN-45 subcutaneous xenograft tumor mice (nude mice were purchased from Changzhou Kavens Laboratory Animal Co., Ltd), 5X10⁶ MKN45 cells were subcutaneously implanted into nude mice; and when the volume of the tumor was about 400 mm³, 5 mg/kg Cy5-P1-AI was injected via a tail vein, and its tissue distribution was analyzed using a living imaging instrument. The results are as shown in FIG. 11. FIG. 11 shows the living imaging results of Cy5-P1-AI in tumor-bearing mice. In the figure, a circle area is a tumor site.

The results show that it can be clearly observed that Cy5-P1-AI was enriched in cancer sites highly expressing c-Met.

### Example 9: P1-AI alanine scanning analysis

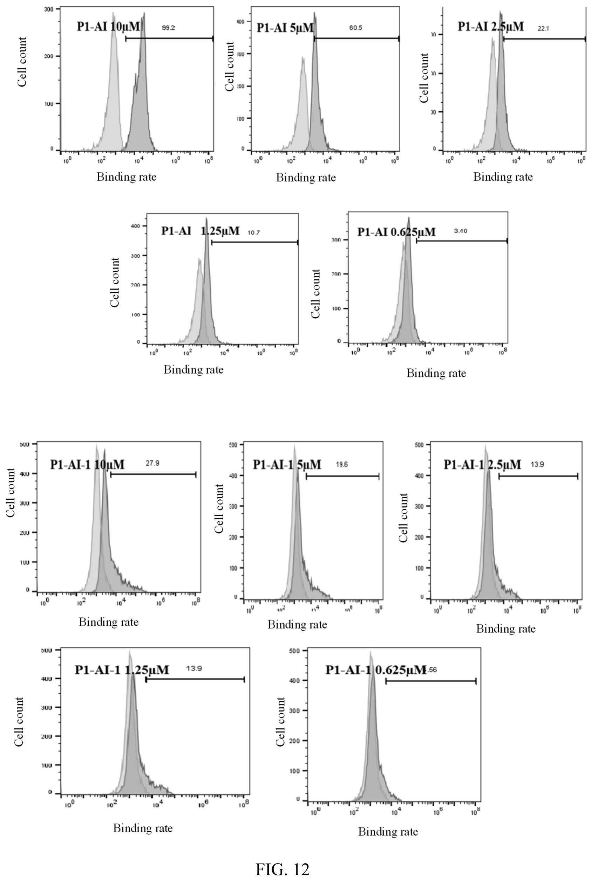

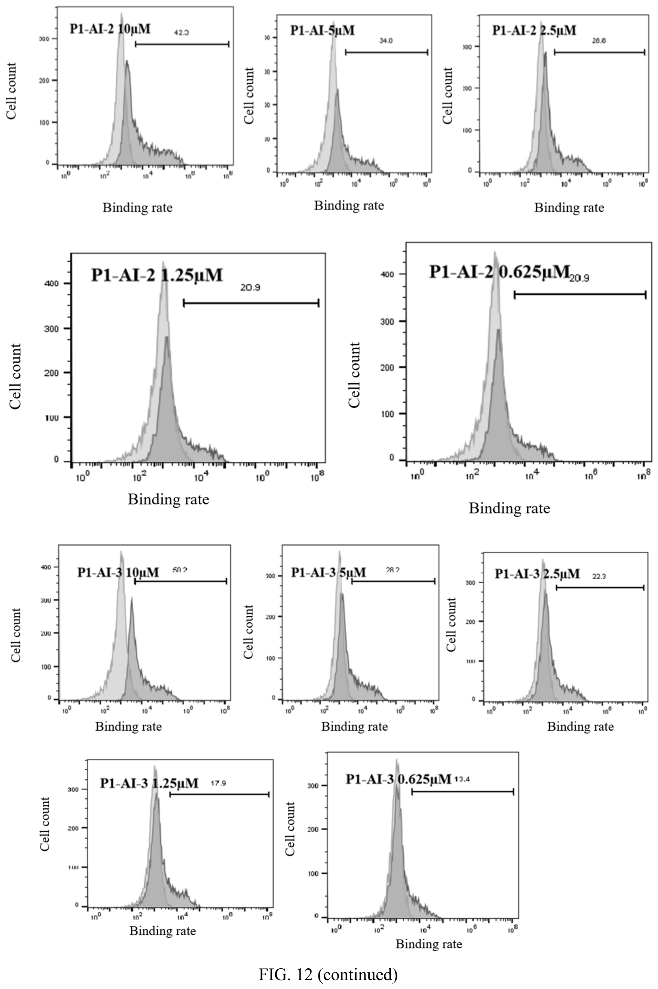

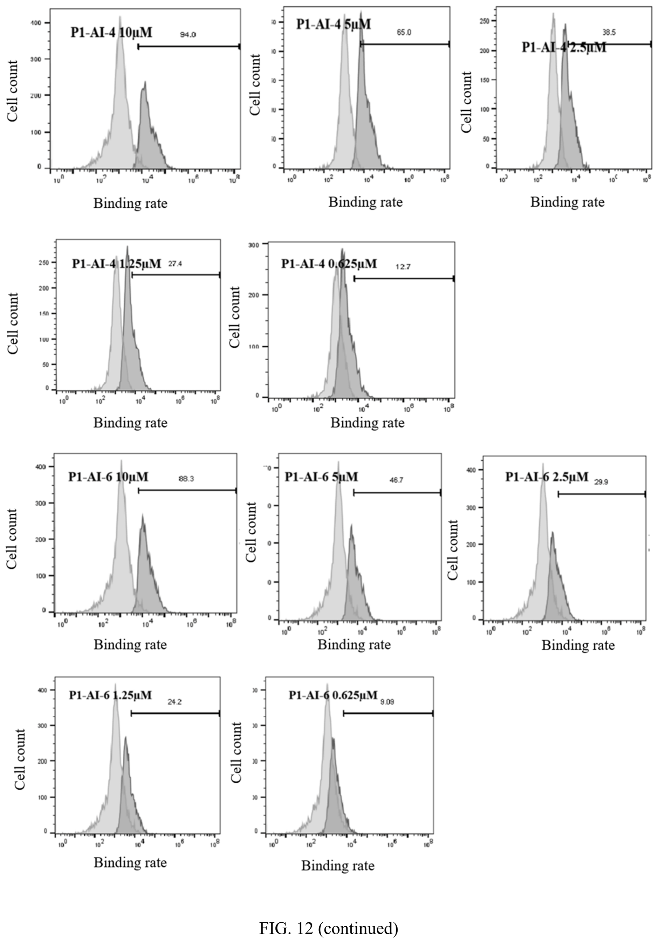

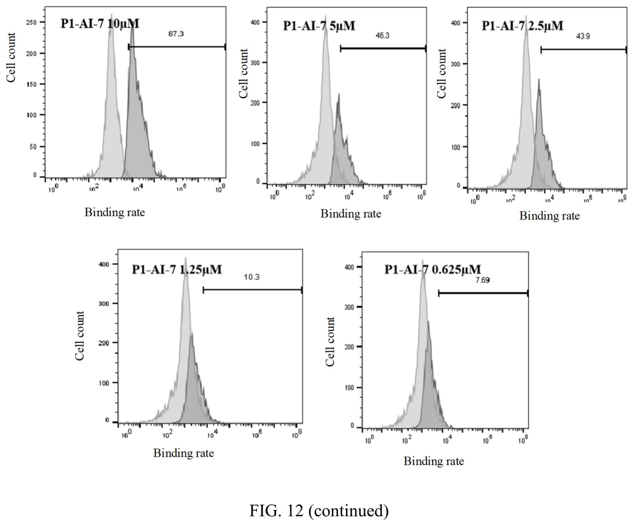

By alanine scanning, P1-AI was used as a starting peptide to generate peptides listed in Table 4. The MKN-45 cells cultured in a 1640 culture solution were collected, and washed twice with pre-cooled PBS. 1 mL of 1% BSA solution was added into the washed cells, and then the above mixed system was placed on a rotary mixer and incubated for 30 min at 4°C.Different concentrations (10 µM, 5 µM, 2.5 µM, 1.25 µM and 0.625 µM) of FITC-P1-AI, FITC-P1-AI-1, FITC-P1-AI-2, FITC-P1-AI-3, FITC-P1-AI-4, FITC-P1-AI-6 and FITC-P1-AI-7 peptide solutions were respectively added in the dark to form 7 groups, and then the above mixed systems were placed on the rotary mixer and incubated for 1 h at 4°C in the dark. After the incubation was completed, the mixed system of the cells was centrifuged for 5 min at 800 rpm, and then supernatant was discarded. The cells were washed 1-2 times with pre-cooled PBS, and then the concentration of the washed cells was adjusted to 1×10⁶ cells/mL by using PBS. 3 parallel replicate wells were set up for cells in each group, with 0.5 mL prepared per sample; and a flow cytometer was used to detect the binding of the peptide to the c-Met positive cell MKN-45. FIG. 12 shows the binding curve of the peptide of the present disclosure and the c-Met positive cell MKN-45, wherein the horizontal axis represents a fluorescence signal; and the longitudinal axis represents the quantity of cells, the dark color is a fluorescence chemical shift after addition of a polypeptide, and the light color is a blank control without the peptide.

**Table 4 Sequences of Peptides**

| SEQ ID NO. | Name | Molecular Weight | Sequence |
|---|---|---|---|
| 145 | FITC-P1-AI-1 | 1211.2 | FITC-ASPYANS |
| 146 | FITC-P1-AI-2 | 1280.36 | FITC -RAPYANS |
| 147 | FITC-P1-AI-3 | 1270.32 | FITC -RSAYANS |
| 148 | FITC-P1-AI-4 | 1204.26 | FITC-RSPAANS |
| 149 | FITC-P1-AI-6 | 1253.33 | FITC-RSPYAAS |
| 150 | FITC-P1-AI-7 | 1280.36 | FITC-RSPYANA |
| 1 | FITC-P1-AI | 1296.36 | FITC-RSPYANS |

FIG. 12 shows that compared with P1-AI, P1-AI-4 exhibits the increased binding affinity to c-Met positive cell MKN45; the binding affinity of P1-AI-6 and P1-AI-7 to c-Met positive cell MKN45 is similar to that of P1-AI; and compared with P1-AI, the binding affinity of P1-AI-1, P1-AI-2 and P1-AI-3 to c-Met positive cell MKN45 is reduced. It can be seen through sequence alignment that the first three sites R, S and P of the P1-AI peptide sequence are key sites that can maintain the ability of binding to c-Met.

In conclusion, the binding affinity of P1-AI, P1-AI-4, P1-AI-6 and P1-AI-7 to c-Met is superior to that of CM7-1.

### Example 10: Solubility of Drug-Peptide Conjugate

Preparation of a camptothecin (CPT) and P1-AI conjugate (CPT-P1-AI), a docetaxel (DOC) and P1-AI conjugate (DOC-P1-AI) and a paclitaxel (PTX) and P1-AI conjugate (PTX-P1-AI): 1.20 g of PTX, CPT or DOC reacted with 0.21 g of succinic anhydride (Anhui Zesheng Technology Co., Ltd.) for 3 h at room temperature to obtain butyric acid-paclitaxel, butyric acid-camptothecin and butyric acid-docetaxel. 0.88 g of butyric acid-paclitaxel, butyric acid-camptothecin or butyric acid-docetaxel reacted with 0.53 g of EDC (Anhui Zesheng Technology Co., Ltd.) /0.21 g of NHS (Shanghai Aladdin Biochemical Technology Co., Ltd.) dissolved in DCM for 9 h at room temperature to obtain a butyric acid-paclitaxel-NHS activated ester, a butyric acid-camptothecin-NHS activated ester or a butyric acid-docetaxel-NHS activated ester. 0.51 g of one of the above three activated esters and 0.58 g of P1-AI polypeptide were dissolved in a solvent (water: N-methylpyrrolidone=1:2.5, V:V) respectively, triethylamine was added to adjust the pH to be weak alkaline, and then the above materials reacted for 4 h at room temperature to obtain CPT-P1-AI, DOC-P1-AI and PTX-P1-AI.

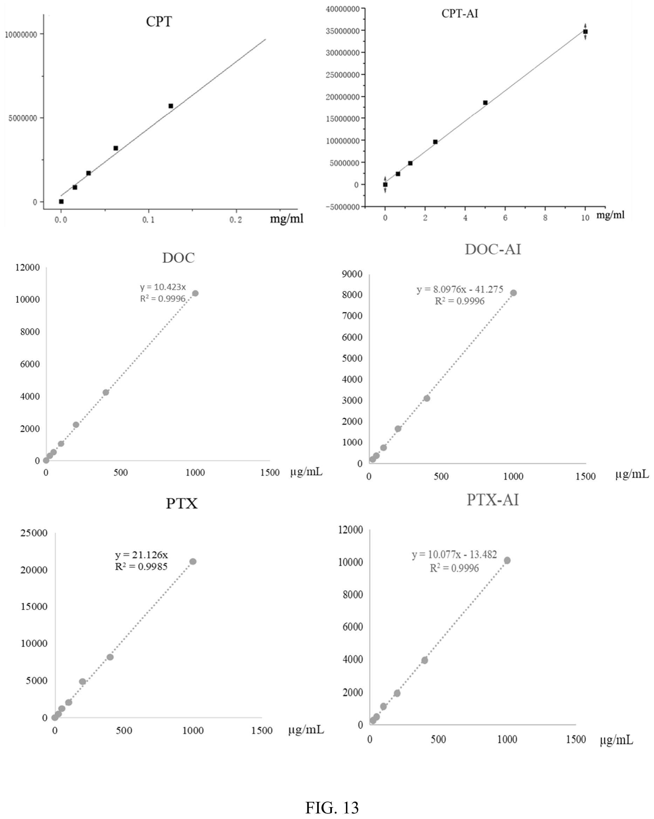

Preparation of the series standard solutions of CPT, CPT-P1-AI, DOC, DOC-P1-AI , PTX and PTX-P1-AI: each 2 mg of CPT, CPT-P1-AI, DOC, DOC-P1-AI, PTX and PTX-P1-AI were weighed and added into a 2 mL volumetric flask, then methanol was added into the flask until the scale mark was reached, and then the solutions were evenly shaken to obtain a 1 mg/mL stock solution.25 µL, 50 µL, 100 µL, 200 µL, 300 µL and 400 µL of the above stock solutions were respectively and precisely measured and added into a 1 ml volumetric flask, then methanol was added into the flask until the scale mark was reached, so as to respectively obtain 25 µg/ml, 50 µg/ml, 100 µg/ml, 200 µg/ml, 300 µg/ml and 400 µg/ml series standard solutions. The peak areas of the series standard solutions were detected via HPLC (mobile phase: 0.065% TFA water + 0.05% TFA; chromatographic column: YMC-TriartC18; wavelength: 220 nm), so as to construct a concentration-peak area standard curve, as shown in FIG. 13. In FIG. 13, the horizontal axis represents the concentration, and the longitudinal axis represents the peak area.

Supersaturated aqueous solutions of CPT, CPT-P1-AI, DOC, DOC-P1-AI, PTX and PTX-P1-AI were prepared, subjected to ultrasonic treatment for 15 min, and filtered using an organic filter membrane and transferred into a liquid-phase vial. The peak area of the sample was detected via HPLC, and the solubility value of the sample in water was calculated by the concentration-peak area standard curve. The results are seen in Table 5.

**Table 5 Solubility Value of Sample in Water**

| Sample | CPT | CPT-P1-AI | DOC | DOC-P1-AI | PTX | PTX-P1-AI |
|---|---|---|---|---|---|---|
| Solubility (mg/mL) | 8.4×10⁻⁵ | 913.37 | 2.74×10⁻⁵ | 4.43 | 1.2×10⁻⁴ | 5.56×10⁻³ |
| Solubility increasing fold of drug-peptide conjugate compared with drug | - | 1.09×10⁷ | - | 1.62×10⁵ | - | 46.33 |

It can be seen from FIG. 13 that the concentration-peak area standard curve fitting degrees of CPT, CPT-P1-AI, DOC, DOC-P1-AI, PTX and PTX-P1-AI are good. It can be seen from Table 5 that CPT, DOC and PTX are barely dissolved in water, their solubilities in water are 8.4×10⁻⁵, 2.74×10⁻⁵ and 1.2×10⁻⁴ mg/mL, respectively. CPT-P1-AI has good solubility in water (913.37 mg/mL), which shows 1.09×10⁷ fold increase compared with positive drug CPT; the solubility of DOC-P1-AI in water is 4.43 mg/mL, which shows a 1.62×10⁵ fold increase compared with that of positive drug DOC; and the solubility of PTX-P1-AI in water shows a 46.33 fold increase compared with that of positive drug PTX.

A compound SNG-1005 formed by coupling a PTX molecule with 19 amino acid peptides Angiopep-2 has completed Phase III clinical trial. However, SNG-1005 is still insoluble after contacting with water, leading to great difficulties in batch production and the forced delay of relevant clinical trials. Machulkin et al. conjugated a PSMA-targeting ligand with DOC via a linker dipeptide to obtain PSMA-DOC, and the solubility of PSMA-DOC in water was 130 µg/mL (Machulkin AE, Uspenskaya AA, Zyk NY, et al. PSMA-targeted small-molecule docetaxel conjugate: Synthesis and preclinical evaluation. Eur J Med Chem. 2022;227: 113936.), the application of PSMA-DOC is still greatly limited. The solubility of the polypeptide conjugate DOC-P1-AI of the present application in water was 4.43mg/mL, which was increased by 1.62×10⁵ fold compared with that of DOC single drug, thereby greatly improving the application of DOC. The poor water solubility of CPT is unfavorable for drug absorption. Currently, researchers have overcome this defect through nano- and micro-formulation systems, but these methods suffer from problems such as low drug loading capacity of the formulation systems and low solvent solubility. The chemical group modification of camptothecin is also used for improving the water solubility of camptothecin. Fan et al. synthesized 7-ethyl-10-[2-oxo-2-(piperidinyl)ethoxy]camptothecin by attaching saturated carbon atoms to camptothecin. The water solubility of the synthesized camptothecin is about 6.22 µg/mL (FAN S,CAO Y X,LI G Y, et al.F10,a new camptothecin derivative, was identified as a new orally-bioavailable, potent antitumor agent[J].Eur J Med Chem,2020,202:112528.). The solubility still needs to be improved, the water solubility of polypeptide conjugate CPT-P1-AI of the present disclosure was 913.37mg/mL, which was improved by 1.09×10⁷ fold compared with that of CPT single drug, thereby greatly improving the application of CPT. The polypeptide of the present application greatly increases the water solubility of CPT, DOC and PTX.

Although specific embodiments have been described, replacements, modifications, variations, improvements and substantial equivalents of the aforementioned embodiments may exist or be currently unforeseeable to the applicant or other skilled persons in the art. Therefore, the appended claims and claims that may be modified are intended to encompass all the replacements, modifications, variations, improvements and substantial equivalents.

## Claims

1. A peptide targeting c-Met or a derivative thereof, comprising the following formula I or formula II,
(R)-(X1)-(X2)-(X3)-(X4)-(X5)-(X6) Formula I
(Y1)-(Y2)-(Y3)-(Y4)-(A)-(N)-(Y5) Formula II
wherein, X1-X6 each independently represent any one amino acid or a derivative thereof;
Y1-Y5 each independently represent any one amino acid or a derivative thereof, and the formula II does not comprise the sequence (D)-(Q)-(I)-(I)-(A)-(N)-(N) of CM7-1; and
the peptide or derivative thereof has similar or better binding affinity to c-Met relative to CM7-1.

2. The peptide or derivative thereof according to claim 1, wherein X1 represents S, H or E, X2 represents P, I or D, X3 represents any one amino acid or a derivative thereof, X4 represents A, M or C, X5 represents N, C or P, and X6 represents any one amino acid or a derivative thereof;
Y1 represents any one amino acid or a derivative thereof, Y2 represents Q or A, Y3 represents I or A, Y4 represents I or A, Y5 represents N or A, and the formula II does not comprise (D)-(Q)-(I)-(I)-(A)-(N)-(N).

3. The peptide or derivative thereof according to claim 1 or 2, comprising any one sequence selected from SEQ ID NO:1, SEQ ID NOs:3-6, SEQ ID NO:8, SEQ ID NOs:9-144 and SEQ ID NOs:148-150.

4. The peptide or derivative thereof according to claim 1 or 2, wherein the peptide or derivative thereof comprises any one sequence selected from SEQ ID NO: 1, SEQ ID NOs:3-6, SEQ ID NO:8, SEQ ID NOs:9-20, SEQ ID NO:22, SEQ ID NO:28, SEQ ID NO:35, SEQ ID NO:40, SEQ ID NO:48, SEQ ID NO:64, SEQ ID NO:66, SEQ ID NO:70, SEQ ID NO:72, SEQ ID NOs:79-81, SEQ ID NOs:83-85, SEQ ID NO:90, SEQ ID NO:96, SEQ ID NO:105, SEQ ID NO:111, SEQ ID NO:123, SEQ ID NO:125, SEQ ID NO:135, SEQ ID NO:144 and SEQ ID NOs: 148-150.

5. The peptide or derivative thereof according to claim 1, comprising the following formula I,
(R)-(X1)-(X2)-(X3)-(X4)-(X5)-(X6) Formula I
wherein,
X1 is S, X2 is P, X3-6 each independently represent any one amino acid or a derivative thereof, and the peptide or derivative thereof has similar or better binding affinity to c-Met relative to P1-AI.

6. The peptide or derivative thereof according to claim 5, wherein the peptide or derivative thereof comprises any one sequence selected from SEQ ID NOs: 148-150.

7. The peptide or derivative thereof according to any one of claims 1-6, wherein compared with the peptide, the derivative comprises any one or more modifications selected from amino acid modification, conservative amino acid substitution and hydrogen substitution in amino acid residue.

8. A drug-peptide conjugate, comprising the peptide or derivative thereof according to any one of claims 1-7 and a drug, preferably, wherein the peptide or derivative thereof is conjugated with a drug directly or by a linker, preferably, the drug is selected from at least one of cytotoxic agents such as camptothecin, docetaxel and paclitaxel.

9. A pharmaceutical composition, comprising the peptide or derivative thereof according to any one of claims 1-7 or the drug-peptide conjugate according to claim 8, and a pharmaceutically acceptable carrier or salt thereof.

10. Use of the peptide or derivative thereof according to any one of claims 1-7 for increasing the water solubility of a drug, wherein the peptide or derivative thereof is conjugated with the drug directly or indirectly.

11. A method for increasing water solubility of a drug, comprising conjugating the peptide or derivative thereof according to any one of claims 1-7 with a drug directly or indirectly.

12. A method for detecting, preventing or treating a disease, the method comprising administrating to a subject in need thereof a detectably or preventively or therapeutically effective amount of the peptide or derivative thereof according to any one of claims 1-7, the drug-peptide conjugate according to claim 8 or the pharmaceutical composition according to claim 9, preferably, wherein the disease comprises cancers such as solid tumors and/or hematological tumors, preferably, the disease comprises one or more of thyroid cancer, esophageal cancer, lung cancer, non-small cell lung cancer, gastric cancer, liver cancer, cholangiocarcinoma, kidney cancer, pancreatic cancer, bowel cancer, colorectal cancer, bladder cancer, prostate cancer, breast cancer, uterine cancer, endometrial cancer, ovarian cancer, cervical cancer, fallopian tube cancer, skin cancer, brain tumor, glioma, astrocytoma, melanoma, osteosarcoma, sarcoma, lymphoma, Hodgkin lymphoma, non-Hodgkin lymphoma, multiple myeloma, leukemia, acute lymphoblastic leukemia, chronic lymphoblastic leukemia, acute myeloid leukemia and chronic myeloid leukemia.
